# EUROPEAN PATENT SPECIFICATION

(11) **EP 2 829 605 B1**
(45) Date of publication and mention of the grant of the patent: **08.05.2019**
(21) Application number: 13763448.1
(22) Date of filing: 20.02.2013
(51) Int. Cl.: C12N 15/09, C12N 5/10, C12Q 1/02, C12Q 1/34, G01N 33/15, G01N 33/50, G01N 33/68, C07K 14/47, C12Q 1/68, C12N 5/079

(54) **METHOD FOR SCREENING THERAPEUTIC AND/OR PROPHYLACTIC AGENTS FOR ALZHEIMER'S DISEASE**
VERFAHREN ZUM SCREENING VON THERAPEUTIKA UND/ODER PROPHYLAKTIKA FÜR MORBUS ALZHEIMER
PROCÉDÉ DE CRIBLAGE D'AGENTS THÉRAPEUTIQUE ET/OU PROPHYLACTIQUE POUR LA MALADIE D'ALZHEIMER

(30) Priority: 21.03.2012 JP 2012064094
(43) Date of publication of application: 28.01.2015
(73) Proprietor: Kyoto University, Kyoto-shi, Kyoto 606-8501 (JP)
(72) Inventor: INOUE, Haruhisa, Kyoto-shi Kyoto 606-8501 (JP); TAKAHASHI, Ryosuke, Kyoto-shi Kyoto 606-8501 (JP); KONDO, Takayuki, Kyoto-shi Kyoto 606-8501 (JP); IWATA, Nobuhisa, Nagasaki-shi Nagasaki 852-8521 (JP); ASAI, Masashi, Nagasaki-shi Nagasaki 852-8521 (JP)
(74) Representative: Hoffmann Eitle
(86) International application number: PCT/JP2013/054199
(87) International publication number: WO 2013/140927

(56) References cited:
- WO-A1-2011/108766
- WO-A1-2012/013936
- WO-A2-2007/127273
- JP-A- 2006 314 242
- US-A1- 2011 046 092
- US-A1- 2011 229 441
- T. YAGI ET AL: "Modeling familial Alzheimer's disease with induced pluripotent stem cells", HUMAN MOLECULAR GENETICS, vol. 20, no. 23, 7 September 2011 (2011-09-07), pages 4530-4539, XP055239838, gb ISSN: 0964-6906, DOI: 10.1093/hmg/ddr394
- TAKAMI TOMIYAMA ET AL: "A new amyloid [beta] variant favoring oligomerization in Alzheimer's-type dementia", ANNALS OF NEUROLOGY., vol. 63, no. 3, 1 March 2008 (2008-03-01), pages 377-387, XP055239846, BOSTON, US ISSN: 0364-5134, DOI: 10.1002/ana.21321
- TAKAYUKI KONDO ET AL: "Modeling Alzheimer's Disease with iPSCs Reveals Stress Phenotypes Associated with Intracellular A[beta] and Differential Drug Responsiveness", CELL STEM CELL, vol. 12, no. 4, 1 April 2013 (2013-04-01), pages 487-496, XP055239837, AMSTERDAM, NL ISSN: 1934-5909, DOI: 10.1016/j.stem.2013.01.009
- CHOI S.H. ET AL.: 'iPSCs to the rescue in Alzheimer's research.' CELL STEM CELL. vol. 10, no. 3, 02 March 2012, pages 235 - 236, XP028402787
- TOMIYAMA T. ET AL.: 'A mouse model of amyloid beta oligomers: their contribution to synaptic alteration, abnormal tau phosphorylation, glial activation, and neuronal loss in vivo.' J NEUROSCI. vol. 30, no. 14, 2010, pages 4845 - 4856, XP055168255
- TAKAMI TOMIYAMA: 'APP E693DELTA Hen'i to Abeta Oligomer Kasetsu' JOURNAL OF JAPANESE BIOCHEMICAL SOCIETY 2009, pages 3S7A - 2, XP008174848
- NISHITSUJI K. ET AL.: 'The E693Delta mutation in amyloid precursor protein increases intracellular accumulation of amyloid beta oligomers and causes endoplasmic reticulum stress-induced apoptosis in cultured cells.' AM J PATHOL. vol. 174, no. 3, 2009, pages 957 - 969, XP055168257
- ISRAEL M.A. ET AL.: 'Probing sporadic and familial Alzheimer's disease using induced pluripotent stem cells.' NATURE vol. 482, no. 7384, 25 January 2012, pages 216 - 220, XP055168259

## Description

### TECHNICAL FIELD

The present invention relates to a method for screening a therapeutic and/or prophylactic agent for Alzheimer's disease.

### BACKGROUND ART

Alzheimer's disease is one of a protein misfolding disease that exhibits deposition of amyloid β protein (Aβ) in brain, and known as a neurodegenerative disease caused by cytotoxicity due to the Aβ deposition (Non-patent Document 1). Since initiating therapy for Alzheimer's disease as early as possible leads to effective treatment of the disease, development of a method for its early diagnosis is an important task in an aging society.

At present, NINCDS-ADRDA and DSM-IV are employed as clinical diagnostic criteria for Alzheimer's disease. These criteria are excellent for diagnosis of positivity of dementia, but cannot eliminate the possibility that the patient is diagnosed as negative for the disease at an early stage of the onset. Therefore, a definite diagnosis cannot be reached, and, needless to say, it is impossible to make a diagnosis before the onset of the disease. Since Alzheimer's disease has been revealed to be caused by accumulation of Aβ, conventional diagnosis of Alzheimer's disease has been made using as indices a decrease in the Aβ42/Aβ40 ratio and an increase in phosphorylated tau protein (p-tau) in the cerebrospinal fluid, and p-tau/(Aβ42/Aβ40), which is the combination of these indices. However, in many cases, the values of these diagnostic indices rise only after progression of nerve cell death. Therefore, even with these indices, early diagnosis and prediction of the onset of Alzheimer's disease are very difficult. At present, several drugs such as Aricept are available as therapeutic agents for Alzheimer's disease, but any of these only has the action to delay the progression of the disease state. Thus, development of a method for early diagnosis and a therapeutic agent for radical treatment has been demanded.

On the other hand, in the fields of regenerative medicine a technology that enables conversion of a cell convenient as a biomaterial into a cell of a desired type has been demanded, and recently, mouse and human induced pluripotent stem cells (iPS cells) were established in succession. Yamanaka et al. introduced four genes, that is, Oct3/4, Sox2, Klf4 and c-Myc, into human skin-derived fibroblasts and thereby succeeded in establishment of iPS cells (Patent Document 1 and Non-patent Document 2). Since the thus obtained iPS cells can be prepared using cells derived from a patient to be treated and then allowed to differentiate into cells of various tissues, they are thought to be capable of reproducing the diseased state *in vitro.* In fact, it has been reported that iPS cells derived from a patient with familial Alzheimer's disease having a mutation in presenilin were prepared by using the above method, and that the cells were successfully induced to differentiate into nerve cells in which extracellular secretion of Aβ42 is increased (Non-patent Document 3).

On the other hand, in a clinical study in which extracellular Aβ was removed by utilizing the immune response, extracellular Aβ decreased but no amelioration of clinical symptoms could be found (Non-patent Document 4). Although there are various opinions on these results, what diagnostic index is useful for screening of therapeutically effective drugs is unknown.

Recently, familial Alzheimer's disease showing a mutation (E693Δ) in amyloid precursor protein (APP) has been reported in Japan. However, deposition of Aβ in the brain of the patient has not been found by positron emission tomography (PET) using a compound having an affinity for amyloid as a probe (Non-patent Document 5). Therefore, whether or not deposition of Aβ, such as formation of senile plaques, causes the onset of Alzheimer's disease still remains unclear. Publication WO2012/013936 discloses a method for screening for a therapeutic compound useful in treatment of Alzheimer's disease wherein neurons obtained from DS-iPS (Down Syndrome-iPS) cells are contacted with the test compound and the effect of the compound on the aggregates of amyloid-β within the neurons is analysed.

Nishitsuji, et al 2009 discloses artificial accumulation amyloid-β compounds in immortalized cell lines and discuses that the mutation E693 induces the accumulation of intracellular amyloid beta in neurons.

### PRIOR ART DOCUMENTS

### [Patent Documents]

Patent Document 1: WO 2007/069666

### [Non-patent Documents]

Non-patent Document 1: Bucciantini M, et al., Nature. 416:507-511 (2002)
Non-patent Document 2: Takahashi, K, et al., Cell. 131:861-872 (2007)
Non-patent Document 3: Yagi, T, et al., Hum Mol Genet. 20:4530-4539 (2011)
Non-patent Document 4: Holmes, C, et al., Lancet 372:16-223 (2008)
Non-patent Document 5: Tomiyama, T, et al., Ann. Neurol. 63, 377-387 (2008)

### SUMMARY OF THE INVENTION

The present invention aims to provide a novel method for screening a therapeutic and/or prophylactic agent for Alzheimer's disease.

As a result of intensive study to solve the above problem, the present inventors succeeded in reproducing the diseased state of Alzheimer's disease in nerve cells or astrocytes whose differentiation was induced from iPS cells derived from somatic cells of a patient with Alzheimer's disease. That is, in these differentiation-induced nerve cells or astrocytes, findings such as intracellular accumulation of Aβ oligomers, increases in the ER stress level, and increases in oxidative stress such as production of reactive oxygen species were observed. Further, the present inventors discovered that addition of existing therapeutic agents or prophylactic agents for Alzheimer's disease causes decreases in the values of these indices, thereby completing the present invention.

That is, the present is defined by the appended claims. The present invention provides the following.
[1] A method for screening a therapeutic and/or prophylactic agent for Alzheimer's disease, the method comprising the steps of:
   (a) bringing a candidate substance into contact with astrocyte(s) derived from an induced pluripotent stem (iPS) cell(s) prepared from a somatic cell(s) of a patient with Alzheimer's disease, or derived from an iPS cell(s) in which APP having deletion mutation of glutamic acid at position 693 has been introduced;
   (b) measuring the amount of Aβ oligomers in the nerve cell(s) or astrocyte(s); and
   (c) selecting the candidate substance as a therapeutic and/or prophylactic agent for Alzheimer's disease if the amount of Aβ oligomers is decreased as compared to a case where the candidate substance is not brought into contact.
[2] The method according to [1], wherein the nerve cell or astrocyte is a cell that accumulates Aβ oligomers.
[3] The method according to [1] or [2], wherein the somatic cell of a patient with Alzheimer's disease is a somatic cell having APP having deletion mutation of glutamic acid at position 693.

The present invention enables screening of a therapeutic and/or prophylactic agent for Alzheimer's disease using a novel tool. Accordingly, the present invention is very useful for early treatment and/or prophylaxis of Alzheimer's disease.

### BRIEF DESCRIPTION OF THE DRAWINGS

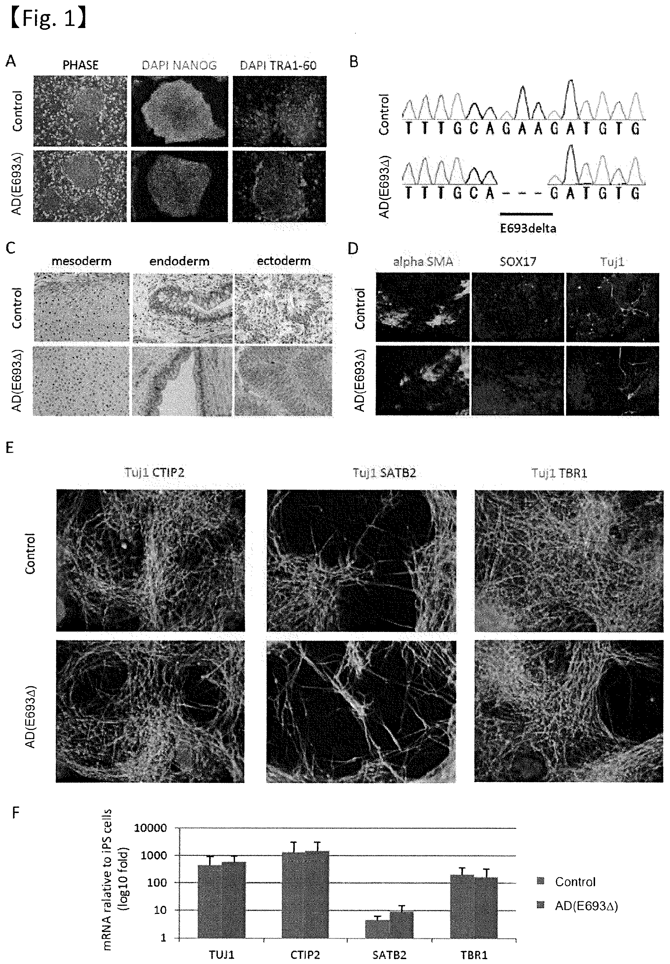

Fig. 1 shows the results of evaluation of the prepared control (control) and AD(E693Δ)-iPSCs, and differentiation induction of these cells into cerebral cortical neurons. (A) Morphology of iPS cells established from somatic cells of a patient with Alzheimer's disease (E693Δ) (phase images), and photographs showing expression of NANOG (green) and TRA1-60 (red), which are markers for pluripotent stem cells. (B) Comparison of the genomic DNA sequence between control iPS cells (Control) and iPS cells established from somatic cells of a patient with Alzheimer's disease (AD(E693Δ)). In the iPS cells derived from somatic cells of a patient with Alzheimer's disease, E693 is homozygously deleted (E693Δ). (C) Photographs showing *in vivo* tridermic differentiation (teratoma) of the established iPS cells. The mesoderm shows cartilage; the endoderm shows intestinal tract-like epithelium; and the ectoderm shows a neural tube-like tissue. (D) Photographs showing *in vitro* tridermic differentiation of the established iPS cells. α-smooth muscle actin represents the mesoderm; SOX17 represents the endoderm; and Tuj1 represents the ectoderm. (E) Photographs showing *in vitro* differentiation of the respective types of established iPS cells into cerebral cortical neurons. The nerve cell marker (Tuj1) is colored in green, and the cerebral cortical transcription marker (CTIP2, SATB2 or TBR1) is colored in red. (F) Relative mRNA expression levels of the nerve cell marker and the cerebral cortical expression markers in cerebral cortical neurons whose differentiation was induced from a control (control) and AD(E693Δ)-iPSCs. Each expression level was standardized against the expression level in the iPS cells.

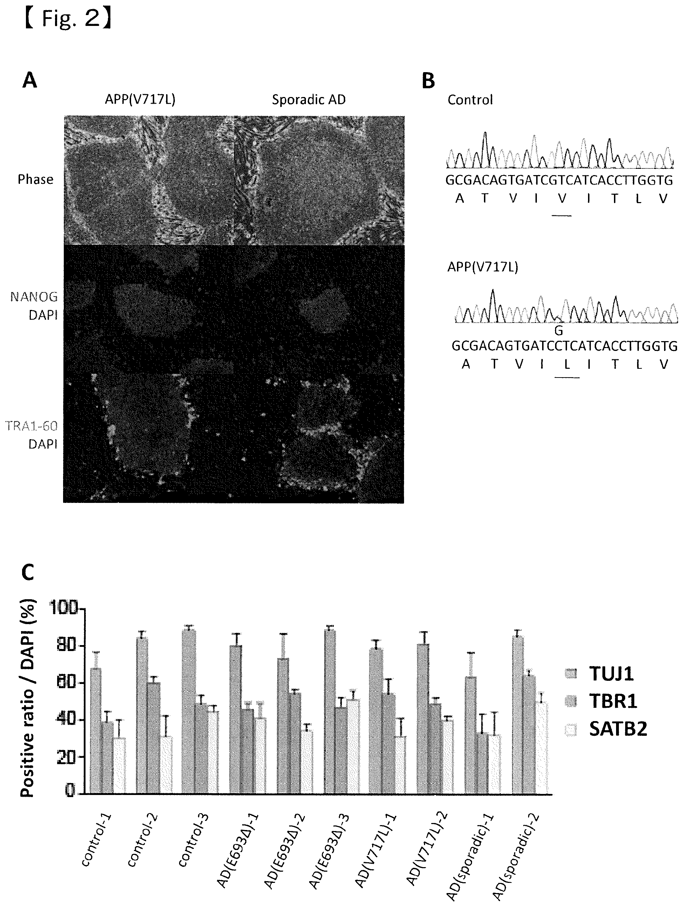

Fig. 2 shows evaluation of the prepared AD(V717L)-iPSCs and sporadic Alzheimer's disease (AD(sporadic))-iPSCs, and the results of differentiation induction of these cells into nerve cells. (A) Morphology of iPS cells established from somatic cells of patients with Alzheimer's disease (V717L and sporadic) (phase images), and photographs showing expression of NANOG (green) and TRA1-60 (red), which are markers for pluripotent stem cells. (B) Comparison of the genomic DNA sequence between control iPS cells (Control) and iPS cells established from somatic cells of a patient with Alzheimer's disease (AD(V717L)). In the iPS cells derived from somatic cells of a patient with Alzheimer's disease (AD(V717L)), APP has a heterozygous mutation of V717 (V717L). (C) The relative protein expression levels of a nerve cell marker (Tuj1) and cerebral cortical transcription markers (TBR1 and SATB2) in cerebral cortical neurons whose differentiation was induced from the control, AD(E693Δ), AD(V717L) or sporadic iPSCs. The graph shows the percentage of the number of cells positive for each marker with respect to the number of nuclei (DAPI).

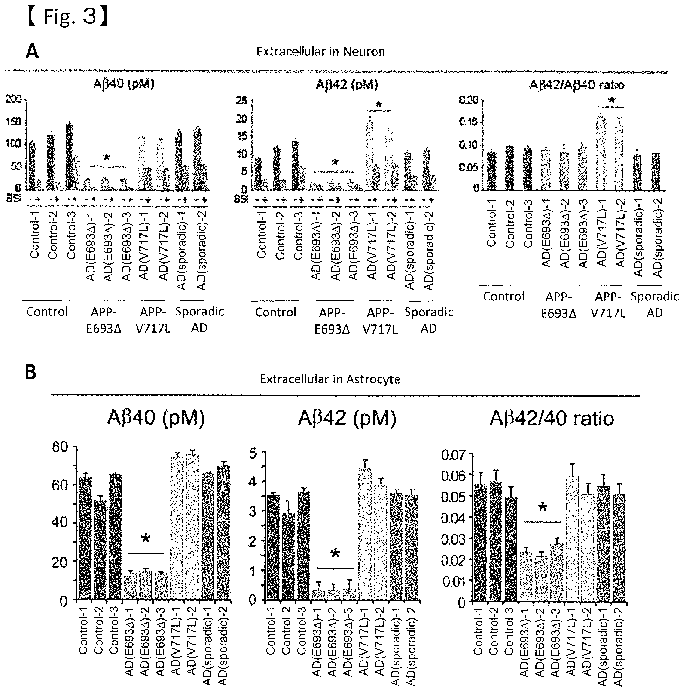

Fig. 3 shows the amount of extracellular Aβ secreted from nerve cells or astrocytes whose differentiation was induced from the control, AD(E693Δ), AD(V717L) or sporadic iPSCs. (A) The amounts of extracellular Aβ40 and Aβ42 secreted from nerve cells, and the Aβ42/Aβ40 ratio. The results obtained for each type of cells with addition (+) or without addition (-) of BSI are shown. The data are shown as mean ± SD (n=3), and * represents significant statistical difference from other astrocytes at p<0.006 (Aβ40 and Aβ42) or p<0.001 (Aβ42/ Aβ40). (B) The amounts of extracellular Aβ40 and Aβ42 secreted from astrocytes, and the Aβ42/Aβ40 ratio. The data are shown as mean ± S. D. (n=3), and * represents significant statistical difference from other astrocytes at p<0.006 (Aβ40 and Aβ42) or p<0.001 (Aβ42/ Aβ40).

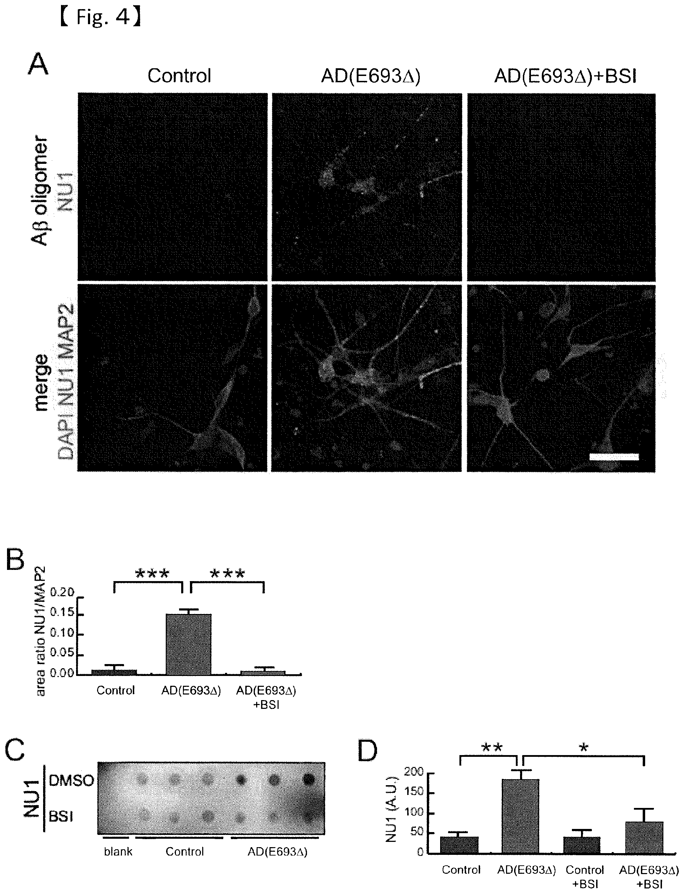

Fig. 4 shows accumulation of Aβ oligomers in nerve cells whose differentiation was induced from iPS cells derived from somatic cells of a patient with Alzheimer's disease (AD(E693Δ)). (A) Photographs showing localization of Aβ oligomers in nerve cells (control or AD(E693Δ)) whose differentiation was induced from iPS cells. Nerve cells whose differentiation was induced from iPS cells (MAP2-positive cells) are shown in red, and Aβ oligomers detected with an Aβ oligomer-specific antibody, NU-1 monoclonal antibody are shown in green. Aβ oligomers were highly accumulated in the nerve cells derived from an AD patient (AD(E693Δ)), but hardly accumulated in the control nerve cells. Further, the β-secretase inhibitor (BSI) decreased Aβ oligomers accumulated in the nerve cells derived from an AD patient (AD(E693Δ)). (B) Accumulation of Aβ oligomers quantified as the NU-1-positive area (%) in the MAP2-positive area. The data are shown as mean ± SD (n=3/group). (C) A photograph showing an image of a dot blot analysis using the NU-1 antibody. (D) A graph obtained by quantification of the obtained image. The data are shown as mean ± SD (n=3/group). BSI decreased Aβ oligomers accumulated in the nerve cells derived from an AD patient (AD(E693Δ)). * represents statistical significance at p<0.05.

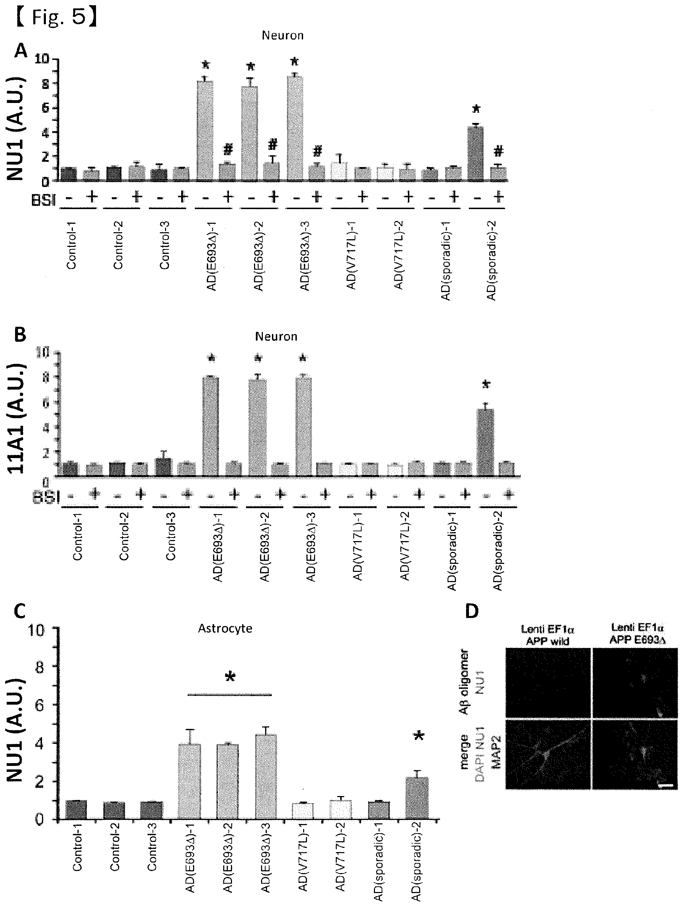

Fig. 5 shows the amount of accumulated Aβ oligomers. (A) A graph showing the amount of Aβ oligomers accumulated in nerve cells whose differentiation was induced from iPS cells (control (control), AD(E693Δ), AD(V717L) and AD(sporadic)), with addition (+) or without addition (-) of BSI. The amounts of accumulated Aβ oligomers were obtained by quantification of the results of a dot plot analysis using an NU1 antibody. The data are shown as mean ± SD (n=3/group). * represents significant statistical difference from other nerve cells at p<0.005, and # represents significant statistical difference from the corresponding nerve cells treated without addition of BSI at p<0.005. (B) A graph showing the amount of Aβ oligomers accumulated in nerve cells whose differentiation was induced from iPS cells (control, AD(E693Δ), AD(V717L) and AD(sporadic)), with addition (+) or without addition (-) of BSI. The amounts of accumulated Aβ oligomers were obtained by quantification of the results of a dot plot analysis using a 11A1 antibody. The data are shown as mean ± SD (n=3/group). * represents significant statistical difference from other nerve cells at p<0.005. (C) A graph showing the amount of Aβ oligomers accumulated in astrocytes (control, AD(E693Δ), AD(V717L) and AD(sporadic)) whose differentiation was induced from iPS cells. The amounts of accumulated Aβ oligomers were obtained by quantification of the results of a dot plot analysis using an NU1 antibody. The data are shown as mean ± SD (n=3/group). * represents significant statistical difference from other astrocytes at p<0.001. (D) Photographs showing localization of Aβ oligomers in nerve cells with wild-type APP expressed by a lentivirus and nerve cells with APP-E693Δ expressed by a lentivirus. Nerve cells whose differentiation was induced from iPS cells (MAP2-positive cells) are shown in red, and Aβ oligomers detected with an Aβ oligomer-specific antibody NU-1 are shown in green.

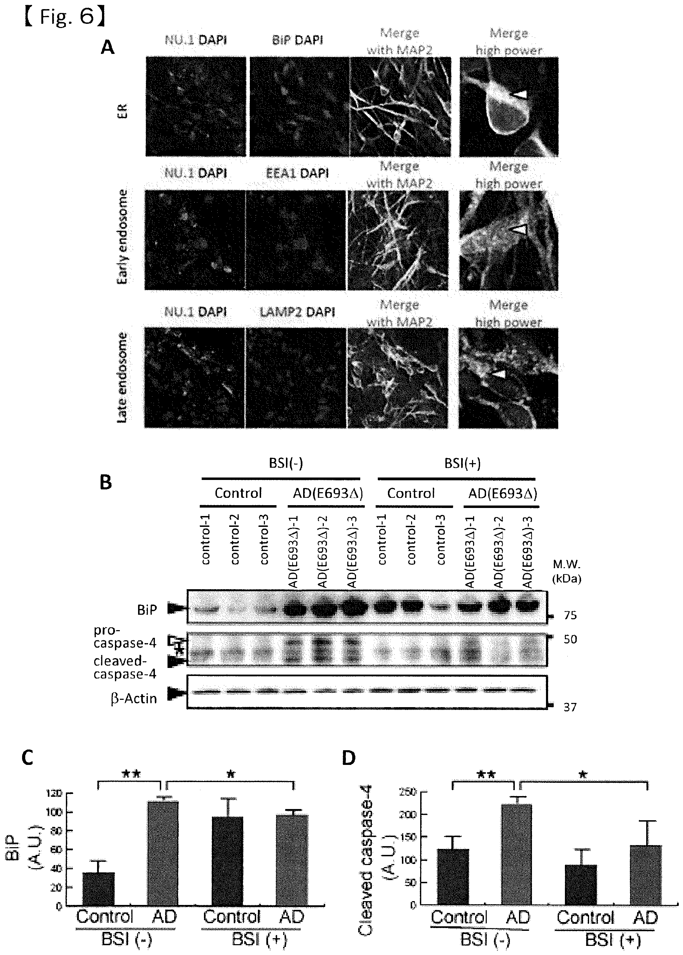

Fig. 6 shows accumulation of Aβ oligomers and the ER stress response in nerve cells whose differentiation was induced from iPS cells derived from somatic cells of a patient with Alzheimer's disease (AD(E693Δ)). (A) Photographs showing localization of Aβ oligomers (green) in organelles (red). BiP represents ER; EEA1 represents early endosomes; and LAMP2 represents late endosomes/lysosomes. The Aβ oligomers were detected with an NU-1 monoclonal antibody. (B) Photographs showing the results of Western blotting analysis of ER stress markers (BiP and activated caspase 4) in nerve cells whose differentiation was induced from iPS cells (control and AD(E693Δ)), in the presence or absence of BSI. (C) Quantification data of BiP. The data are shown as mean ± SD (n=3/group). (D) Quantification data of activated (cleaved) caspase 4. The data are shown as mean ± SD (n=3/group). In this panel, * represents p<0.05, and ** represents p<0.01 (Tukey test).

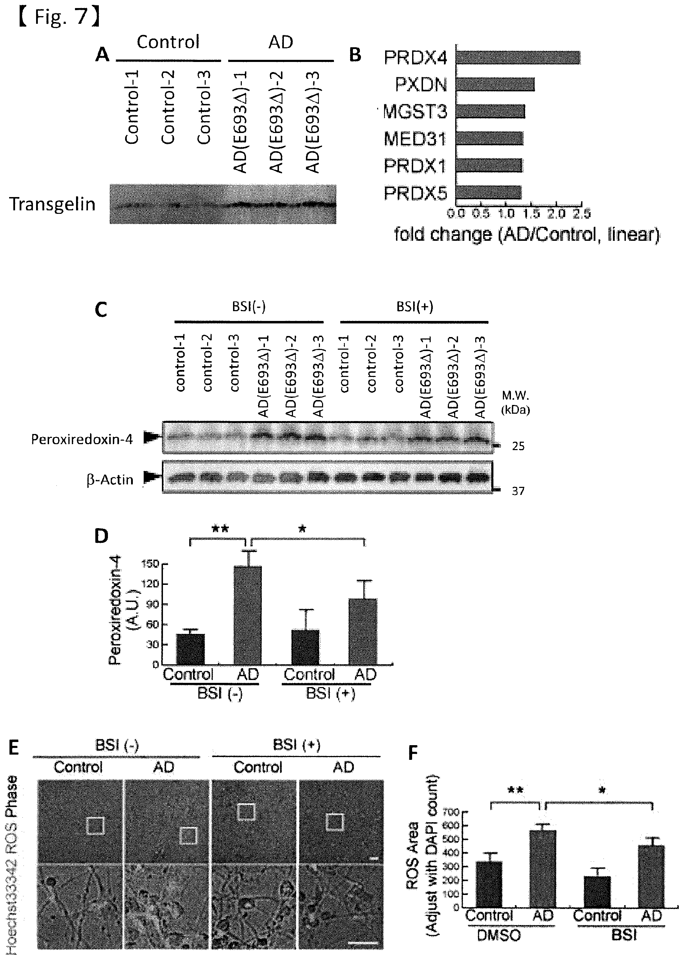

Fig. 7 shows the results of analysis of other markers in nerve cells whose differentiation was induced from iPS cells derived from somatic cells of a patient with Alzheimer's disease (AD(E693Δ)). (A) A photograph showing the results of Western blotting analysis of transgelin. (B) The expression levels of peroxidative activity-related genes in AD relative to the control in gene ontology analysis. (C) Photographs showing the results of Western blotting analysis of peroxiredoxin-4 in nerve cells (control and AD(E693Δ)) whose differentiation was induced from iPS cells, in the presence or absence of BSI. (D) Quantification data of peroxiredoxin-4. The data are shown as mean ± SD (n=3/group). In this panel, * represents p<0.05, and ** represents p<0.01 (Tukey test). (E) Photographs showing typical examples of stained images of reactive oxygen species (ROS) (green) and Hoechst 33342 (blue). (F) A graph showing the results of quantification of the ROS-positive area relative to the DAPI count. The data are shown as mean ± SD (n=3/group). In this panel, * represents p<0.05, and ** represents p<0.01 (Tukey test).

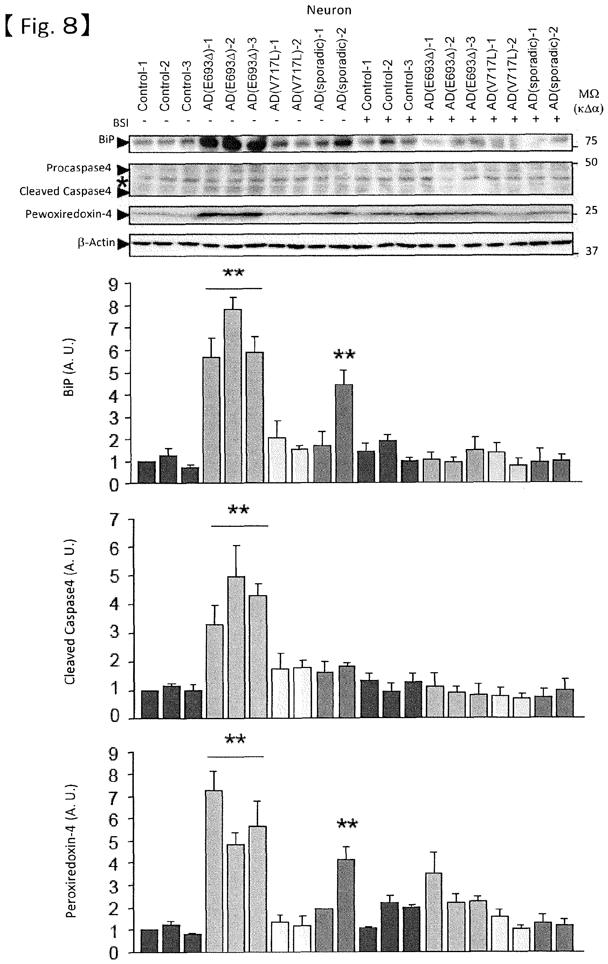

Fig. 8 shows photographs showing the results of Western blot analysis of ER stress marker genes (BiP and activated caspase 4) and an oxidative stress marker gene (peroxiredoxin-4) in nerve cells whose differentiation was induced from iPS cells derived from somatic cells of a patient with Alzheimer's disease (control, AD(E693Δ), AD(V717L) or AD(sporadic)), in the presence or absence of BSI. In BiP and peroxiredoxin-4, significantly higher levels of expression were observed for AD(E693Δ)-1, AD(E693Δ)-2, AD(E693Δ)-3 and AD(sporadic)-2 in the absence of BSI (**p<0.005). In activated caspase 4, significantly higher levels of expression were observed for AD(E693Δ)-1, AD(E693Δ)-2 and AD(E693Δ)-3 in the absence of BSI (**p<0.005).

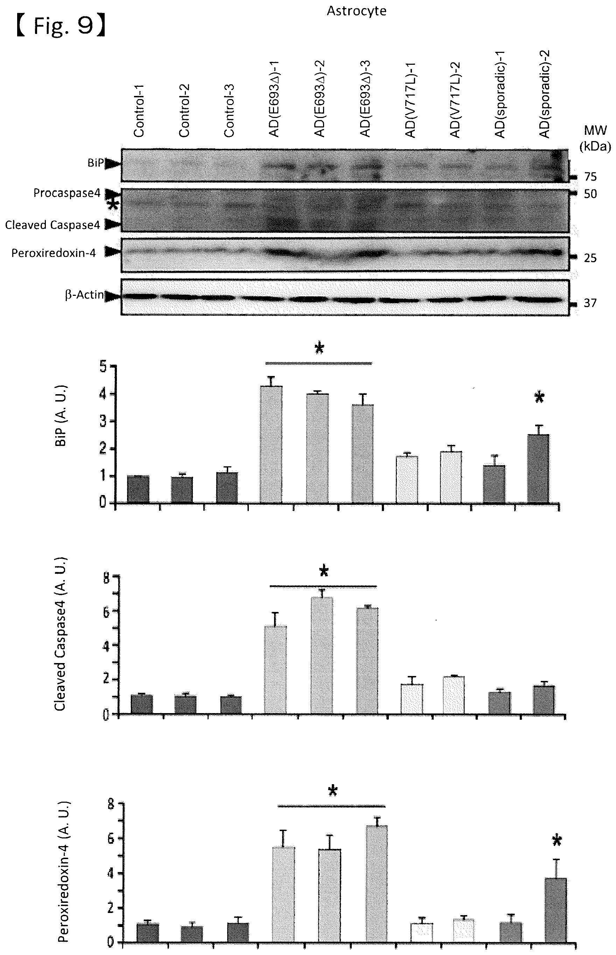

Fig. 9 shows photographs showing the results of Western blot analysis of ER stress marker genes (BiP and activated caspase 4) and an oxidative stress marker gene (peroxiredoxin-4) in astrocytes whose differentiation was induced from iPS cells derived from somatic cells of a patient with Alzheimer's disease (control, AD(E693Δ), AD(V717L) or AD(sporadic)). In BiP and peroxiredoxin-4, significantly higher levels of expression were observed for AD(E693Δ)-1, AD(E693Δ)-2, AD(E693Δ)-3 and AD(sporadic)-2 in the absence of BSI. In activated caspase 4, significantly higher levels of expression were observed for AD(E693Δ)-1, AD(E693Δ)-2 and AD(E693Δ)-3 in the absence of BSI.

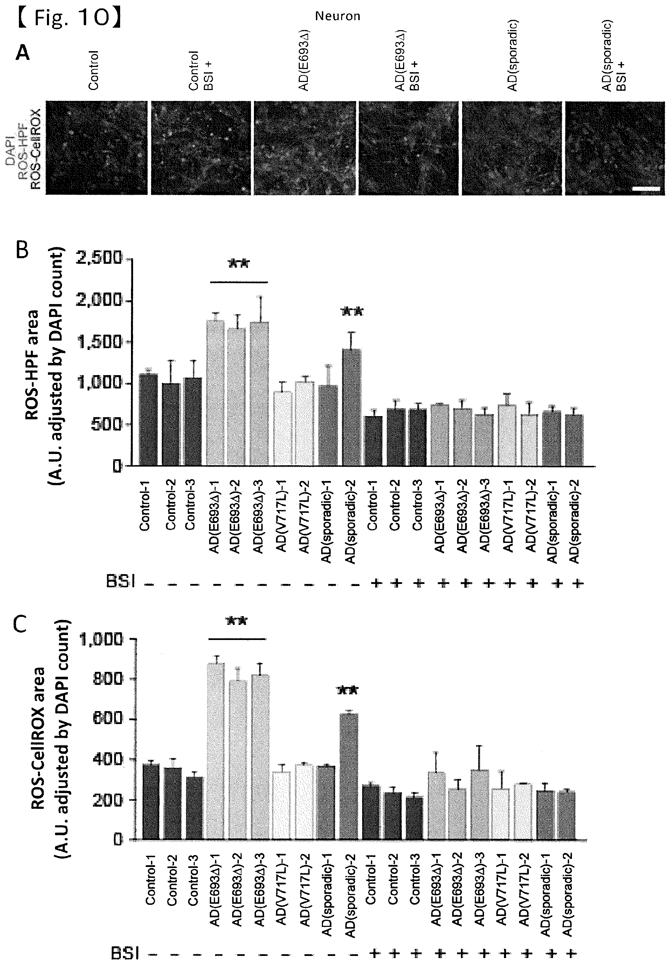

Fig. 10 shows the results of measurement of the amount of reactive oxygen species (ROS) in nerve cells whose differentiation was induced from iPS cells derived from somatic cells of a patient with Alzheimer's disease (control, AD(E693Δ), AD(V717L) or AD(sporadic)), in the presence or absence of BSI. (A) Photographs showing typical examples of staining with 3'-(p-hydroxyphenyl) fluorescein (HPF) (green), staining with CellROX (red) and staining with DAPI (blue) in each type of nerve cells (control, AD(E693Δ) or AD(sporadic)). (B) A graph showing the results of quantification of the ROS-positive area detected with HPF relative to the DAPI count. The data are shown as mean ± SD (n=3/group). In this panel, ** represents p<0.001. (C) A graph showing the results of quantification of the ROS-positive area detected with CellROX relative to the DAPI count. The data are shown as mean ± SD (n=3/group). In this panel, ** represents p<0.001.

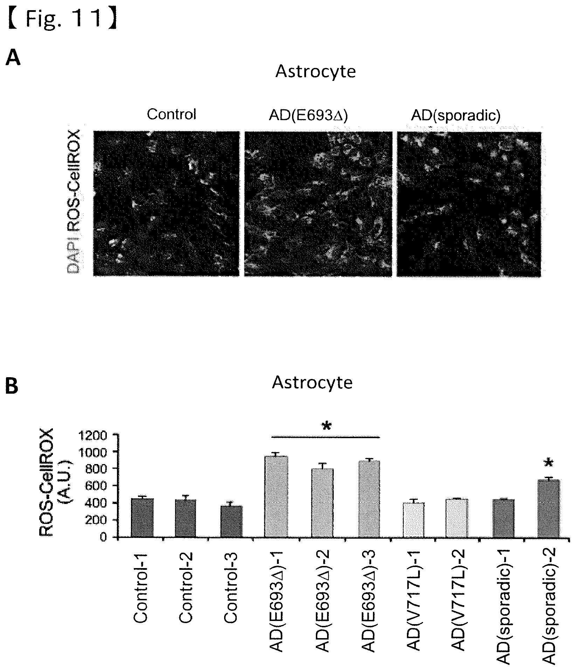

Fig. 11 shows the results of measurement of the amount of reactive oxygen species (ROS) in astrocytes whose differentiation was induced from iPS cells derived from somatic cells of a patient with Alzheimer's disease (control, AD(E693Δ), AD(V717L) or AD(sporadic)). (A) Photographs showing typical examples of staining with CellROX (white) and staining with DAPI (blue) in each type of astrocytes (control, AD(E693Δ) or AD(sporadic)). (B) A graph showing the results of quantification of the ROS-positive area detected with CellROX. The data are shown as mean ± SD (n=3/group).

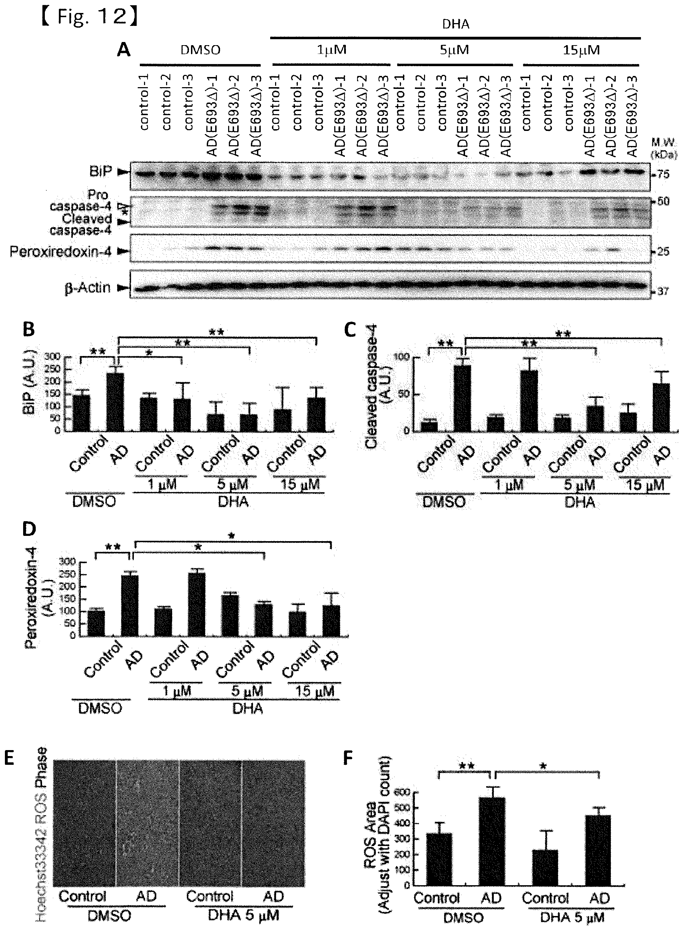

Fig. 12 shows data obtained by observing reduction of the ER stress caused by Aβ oligomer by addition of Docosahexaenoic acid (DHA). (A) Photographs showing the results of Western blot analysis of BiP, activated caspase 4 and peroxiredoxin-4 in a lysate of nerve cells derived from each type of iPS cells (control or AD(E693Δ)), after addition of DHA or solvent DMSO to the medium at each concentration (1 µM, 5 µM or 15 µM). (B) Data obtained by densitometric quantification of the results of Western blot analysis of BiP. The data are shown as mean ± SD (n=3/group). In this panel, * represents p<0.05, and ** represents p<0.01 (Tukey test). (C) Data obtained by densitometric quantification of the results of Western blot analysis of activated caspase 4. The data are shown as mean ± SD (n=3/group). In this panel, ** represents p<0.01 (Tukey test). (D) Data obtained by densitometric quantification of the results of Western blot analysis of peroxiredoxin-4. The data are shown as mean ± SD (n=3/group). In this panel, * represents p<0.05, and ** represents p<0.01 (Tukey test). (E) Photographs showing typical examples of stained images of reactive oxygen species (ROS) (green) and Hoechst 33342 (blue) observed after addition of DHA. (F) A graph showing the results of quantification of the ROS-positive area relative to the DAPI count. The data are shown as mean ± SD (n=3/group). In this panel, * represents p<0.05, and ** represents p<0.01 (Tukey test).

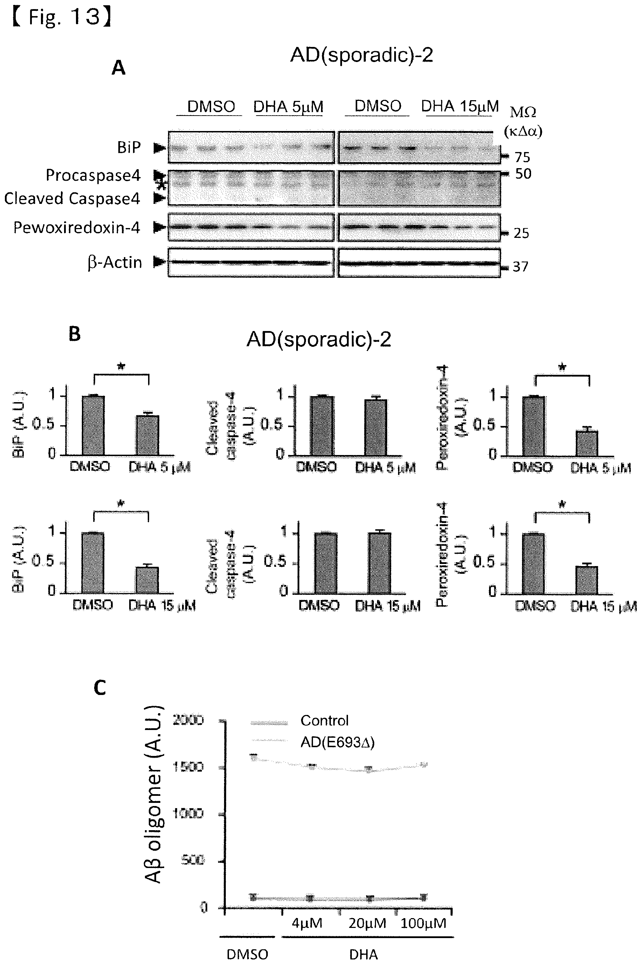

Fig. 13 shows the results of addition of DHA to nerve cells whose differentiation was induced from iPS cells of a patient with Alzheimer's disease. (A) Photographs showing the results of Western blot analysis of BiP, activated caspase 4 and peroxiredoxin-4 in a lysate of nerve cells derived from each type of iPS cells (control or AD(sporadic)-2), after addition of 5 µM or 15 µM DHA or solvent DMSO to the medium. (B) Data obtained by densitometric quantification of the results of Western blot analysis. The data are shown as mean ± SD (n=3/group). In this panel, * represents p<0.005. (C) Data obtained by densitometric quantification of the results of dot blot analysis of Aβ oligomers using an NU1 antibody. The data are shown as mean ± SD (n=3/group).

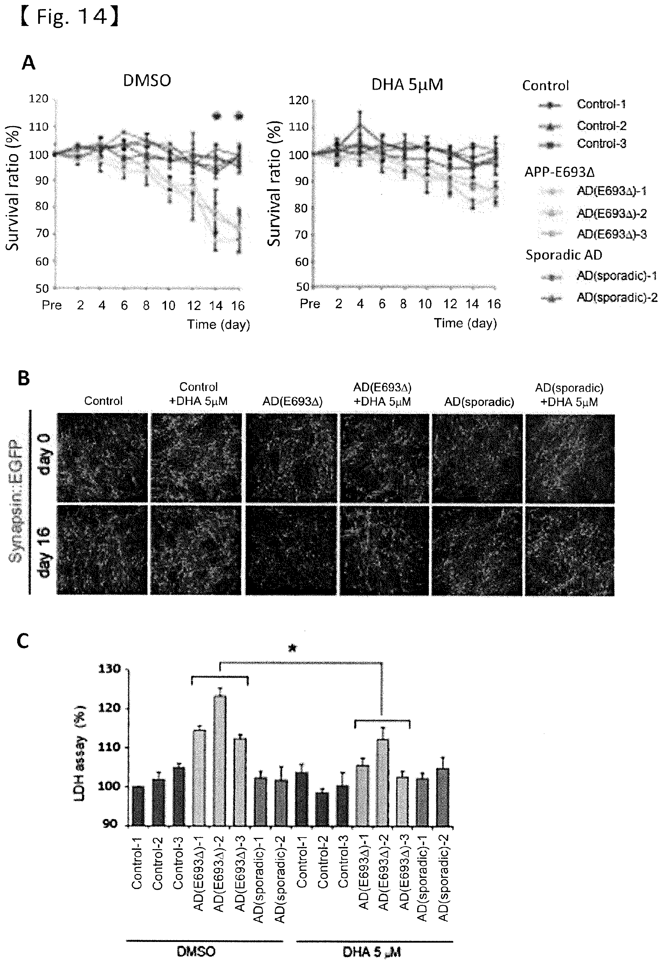

Fig. 14 shows the survival rate of nerve cells whose differentiation was induced from iPS cells derived from somatic cells of a patient with Alzheimer's disease (control, AD(E693Δ) and AD(sporadic)). (A) The survival rates of the control and AD nerve cells in the presence or absence of DHA. The rates were calculated from the numbers of EGFP-positive cells induced by the synapsin I promoter. The data are shown as mean ± SD (n=3/group). In this panel, * represents p<0.001. (B) Photographs showing typical examples of fluorescence images of nerve cells having EGFP (green) induced by the synapsin I promoter in each type of nerve cells (control, AD(E693Δ) and AD(sporadic)). (C) The LDH activity in each type of nerve cells (control, AD(E693Δ) and AD(sporadic)) on Day 16 after DHA treatment. The data are shown as mean ± SD (n=3/group). In this panel, * represents p<0.05.

### EMBODIMENTS FOR CARRYING OUT THE INVENTION

The present invention provides a method for screening a therapeutic and/or prophylactic agent for Alzheimer's disease, which method comprises the step of bringing a candidate substance into contact astrocytes derived from induced pluripotent stem cells (iPS cells) prepared from somatic cells of a patient with Alzheimer's disease.

### Method for Producing iPS Cells

In the present invention, the iPS cells are somatic cell-derived artificial stem cells having properties almost equivalent to those of ES cells such as pluripotency of differentiation and growth ability by self-renewal, and can be prepared by introducing certain specific nuclear reprogramming substances in the form of DNA or protein to somatic cells or by increasing expression of the endogenous mRNAs and proteins of the nuclear reprogramming substances using an agent (K. Takahashi and S. Yamanaka (2006) Cell, 126: 663-676; K. Takahashi et al. (2007) Cell, 131: 861-872; J. Yu et al. (2007) Science, 318: 1917-1920; M. Nakagawa et al. (2008) Nat. Biotechnol., 26: 101-106; WO 2007/069666; and WO 2010/068955). The nuclear reprogramming substances are not restricted as long as these are genes specifically expressed in ES cells, or genes playing important roles in maintenance of the undifferentiated state of ES cells, or gene products thereof. Examples of the nuclear reprogramming substances include Oct3/4, Klf4, Klf1, Klf2, Klf5, Sox2, Sox1, Sox3, Sox15, Sox17, Sox18, c-Myc, L-Myc, N-Myc, TERT, SV40 Large T antigen, HPV16 E6, HPV16 E7, Bmil, Lin28, Lin28b, Nanog, Esrrb, Esrrg and Glis1. These reprogramming substances may be used in combination for establishment of iPS cells. For example, the combination may contain at least one, two or three, preferably four, of the above reprogramming substances.

The nucleotide sequences of the mouse and human cDNAs of the nuclear reprogramming substances described above, and the amino acid sequence information of the proteins encoded by the cDNAs can be obtained by reference to the NCBI accession numbers described in WO 2007/069666, and the mouse and human cDNA sequences and the amino acid sequence information of L-Myc, Lin28, Lin28b, Esrrb, Esrrg and Glis1 can be obtained by reference to the following NCBI accession numbers. Those skilled in the art can prepare a desired nuclear reprogramming substance based on the cDNA sequence or the amino acid sequence information, according to a conventional method.

| Gene name | Mouse | Human |
|---|---|---|
| L-Myc | NM_008506 | NM_001033081 |
| Lin28 | NM_145833 | NM_024674 |
| Lin28b | NM_001031772 | NM_001004317 |
| Esrrb | NM_011934 | NM_004452 |
| Esrrg | NM_011935 | NM_001438 |
| Glis1 | NM_147221 | NM_147193 |

These nuclear reprogramming substances may be introduced into somatic cells in the form of protein by a method such as lipofection, linking to a cell-permeable peptide, or microinjection. Alternatively, the nuclear reprogramming substances may be introduced into somatic cells in the form of DNA by, for example, use of a vector such as a virus, plasmid or artificial chromosome vector; lipofection; use of liposomes; or microinjection. Examples of the virus vector include retrovirus vectors, lentivirus vectors (these are described in Cell, 126, pp. 663-676, 2006; Cell, 131, pp. 861-872, 2007; and Science, 318, pp. 1917-1920, 2007), adenovirus vectors (Science, 322, 945-949, 2008), adeno-associated virus vectors, and Sendai virus vectors (Proc Jpn Acad Ser B Phys Biol Sci. 85, 348-62, 2009). Examples of the artificial chromosome vector include human artificial chromosome (HAC), yeast artificial chromosome (YAC), and bacterial artificial chromosome (BAC, PAC). Examples of the plasmid which may be used include plasmids for mammalian cells (Science, 322: 949-953, 2008). The vector may contain a regulatory sequence(s) such as a promoter, enhancer, ribosome binding sequence, terminator and/or polyadenylation site in order to allow expression of the nuclear reprogramming substance(s). Examples of the promoter include the EF1α promoter, CAG promoter, SRα promoter, SV40 promoter, LTR promoter, CMV (cytomegalovirus) promoter, RSV (Rous sarcoma virus) promoter, MoMuLV (Moloney murine leukemia virus) LTR and HSV-TK (herpes simplex virus thymidine kinase) promoter. Among these, the EF1α promoter, CAG promoter, MoMuLV LTR, CMV promoter, SRα promoter and the like are preferred. The vectors may further contain, as required, a sequence of a selection marker such as a drug resistance gene (e.g., kanamycin-resistant gene, ampicillin-resistant gene or puromycin-resistant gene), thymidine kinase gene or diphtheria toxin gene; a gene sequence of a reporter such as the green-fluorescent protein (GFP), β-glucuronidase (GUS) or FLAG; or the like. Further, in order to remove, after introduction of the above vector into somatic cells, the genes encoding nuclear reprogramming substances, or both the promoters and the genes encoding reprogramming substances linked thereto, the vector may have loxP sequences upstream and downstream of these sequences. Another preferred mode uses a method in which the transgene(s) is/are incorporated into a chromosome(s) using a transposon, and transposase is then allowed to act on the cells using a plasmid vector or adenovirus vector for completely removing the transgene(s) from the chromosome(s). Preferred examples of the transposon include piggyBac, which is a transposon derived from a lepidopteran insect (Kaji, K. et al., (2009), Nature, 458: 771-775; Woltjen et al., (2009), Nature, 458: 766-770; and WO 2010/012077). Further, the vector may contain the origin of lymphotrophic herpes virus, BK virus or Bovine papillomavirus and sequences involved in their replication, such that the vector can replicate without incorporation into the chromosome and exist episomally. Examples of such a vector include vectors containing EBNA-1 and oriP sequences and vectors containing Large T and SV40ori sequences (WO 2009/115295, WO 2009/157201 and WO 2009/149233). Further, in order to introduce a plurality of nuclear reprogramming substances at the same time, an expression vector which allows polycistronic expression may be used. In order to allow the polycistronic expression, the sequences encoding the genes may be linked to each other via IRES or the foot-and-mouth disease virus (FMDV) 2A coding region (Science, 322:949-953, 2008; and WO 2009/092042 2009/152529).

For enhancing the induction efficiency of iPS cells upon the nuclear reprogramming, histone deacetylase (HDAC) inhibitors [for example, low molecular inhibitors such as valproic acid (VPA) (Nat. Biotechnol., 26(7): 795-797 (2008)), trichostatin A, sodium butyrate, MC 1293 and M344; and nucleic acid-type expression inhibitors such as siRNAs and shRNAs against HDAC (e.g., HDAC1 siRNA Smartpool (registered trademark) (Millipore) and HuSH 29mer shRNA Constructs against HDAC1 (OriGene))], DNA methyltransferase inhibitors (e.g., 5'-azacytidine) (Nat. Biotechnol., 26(7): 795-797 (2008)), G9a histone methyltransferase inhibitors [for example, low molecular inhibitors such as BIX-01294 (Cell Stem Cell, 2: 525-528 (2008)); and nucleic acid-type expression inhibitors such as siRNAs and shRNAs against G9a (e.g., G9a siRNA (human) (Santa Cruz Biotechnology))], L-channel calcium agonists (e.g., Bayk8644) (Cell Stem Cell, 3, 568-574 (2008)), p53 inhibitors (e.g., siRNAs and shRNAs against p53) (Cell Stem Cell, 3, 475-479 (2008)), Wnt Signaling activators (e.g., soluble Wnt3a) (Cell Stem Cell, 3, 132-135 (2008)), growth factors such as LIF and bFGF, ALK5 inhibitors (e.g., SB431542) (Nat. Methods, 6: 805-8 (2009)), mitogen-activated protein kinase signaling inhibitors, glycogen synthase kinase-3 inhibitors (PLoS Biology, 6(10), 2237-2247 (2008)), miRNAs such as miR-291-3p, miR-294 and miR-295 (R.L. Judson et al., Nat. Biotech., 27: 459-461 (2009)), and the like may be used in addition to the above-described factors.

Examples of the agent used in the method for increasing expression of the endogenous proteins of nuclear reprogramming substances using an agent include 6-bromoindirubin-3'-oxime, indirubin-5-nitro-3'-oxime, valproic acid, 2-(3-(6-methylpyridin-2-yl)-1H-pyrazol-4-yl)-1,5-naphthyridine, 1-(4-methylphenyl)-2-(4,5,6,7-tetrahydro-2-imino-3(2H)-benzothiazolyl)ethanone HBr (pifithrin-alpha), prostaglandin J2 and prostaglandin E2 (WO 2010/068955).

Examples of the culture medium for induction of the iPS cells include (1) DMEM, DMEM/F12 and DME supplemented with 10 to 15% FBS (these media may further contain LIF, penicillin/streptomycin, puromycin, L-glutamine, non-essential amino acids, β-mercaptoethanol and/or the like, if necessary); (2) culture media for ES cells supplemented with bFGF or SCF, for example, culture media for mouse ES cells (e.g., TX-WES medium, Thromb-X) and culture media for primate ES cells (e.g., culture medium for primate (human and monkey) ES cells (ReproCELL Inc., Kyoto, Japan), mTeSR-1).

Examples of the culture method include a method wherein somatic cells and nuclear reprogramming substances (DNAs or proteins) are brought into contact with each other at 37°C in the presence of 5% CO₂ in DMEM or DMEM/F12 medium supplemented with 10% FBS, and the cells are cultured for about 4 to 7 days, followed by replating the cells on feeder cells (e.g., mitomycin C-treated STO cells or SNL cells) and starting culture in a bFGF-containing culture medium for primate ES cells about 10 days after the contact between the somatic cells and the reprogramming substances, thereby allowing ES cell-like colonies to appear about 30 to about 45 days after the contact, or later. To enhance the induction efficiency of iPS cells, the culture may be carried out under conditions where the concentration of oxygen is as low as 5 to 10%.

As an alternative culture method, the somatic cells may be cultured on feeder cells (e.g., mitomycin C-treated STO cells or SNL cells) in DMEM medium supplemented with 10% FBS (which may further contain LIF, penicillin/streptomycin, puromycin, L-glutamine, non-essential amino acids, β-mercaptoethanol and/or the like, if necessary), thereby allowing ES-like colonies to appear after about 25 to about 30 days of the culture, or later.

During the above culture, the culture medium is replaced with a fresh medium once every day from Day 2 of the culture. The number of the somatic cells used for nuclear reprogramming is not restricted, and usually within the range of about 5×10³ to about 5×10⁶ cells per 100-cm² area on the culture dish.

In cases where a gene containing a drug resistance gene is used as a marker gene, cells expressing the marker gene can be selected by culturing the cells in a culture medium containing the corresponding drug (selection medium). Cells expressing a marker gene can be detected by observation under a fluorescence microscope in cases where the marker gene is the gene of a fluorescent protein; by adding a luminescent substrate in cases where the marker gene is the gene of luciferase; or by adding a coloring substrate in cases where the marker gene is the gene of a coloring enzyme.

The "somatic cells" used in the present specification may be any cells, excluding germ cells, derived from a mammal (e.g., human, mouse, monkey, pig or rat). Examples of the somatic cells include epithelial cells which are keratinized (e.g., keratinized epidermal cells), mucosal epithelial cells (e.g., epithelial cells of the lingual surface), epithelial cells of exocrine glands (e.g., mammary cells), hormone-secreting cells (e.g., adrenomedullary cells), cells for metabolism and storage (e.g., hepatic cells), luminal epithelial cells constituting boundary surfaces (e.g., type I alveolar cells), luminal epithelial cells in the closed circulatory system (e.g., vascular endothelial cells), ciliated cells having a carrying capacity (e.g., tracheal epithelial cells), extracellular matrix-secreting cells (e.g., fibroblasts), contractile cells (e.g., smooth muscle cells), cells involved in the blood system and the immune system (e.g., T lymphocytes), sensory cells (e.g., rod cells), autonomic neurons (e.g., cholinergic neurons), supporting cells of sense organs and peripheral neurons (e.g., satellite cells), nerve cells and glial cells in the central nervous system (e.g., astroglial cells) and pigment cells (e.g., retinal pigment epithelial cells), and progenitor cells (tissue progenitor cells) thereof. The level of differentiation of the cells and the age of the animal from which the cells are collected are not restricted, and either undifferentiated progenitor cells (including somatic stem cells) or terminally-differentiated mature cells may be similarly used as the source of the somatic cells in the present invention. Here, examples of the undifferentiated progenitor cells include tissue stem cells (somatic stem cells) such as neural stem cells, hematopoietic stem cells, mesenchymal stem cells and dental pulp stem cells.

In the present invention, the mammalian individual from which somatic cells are derived is not restricted, and preferably human.

The iPS cells used in the present invention is preferably prepared from somatic cells collected from a patient who is known to be suffering from Alzheimer's disease. More preferably, the iPS cells prepared from somatic cells collected from a patient who is known to be suffering from Alzheimer's disease are cells that accumulate Aβ oligomers upon induction into nerve cells or astrocytes.

In the present invention, Aβ means amyloid β protein, which is a fragment produced by cleavage of amyloid precursor protein (APP) by β- and γ-secretases. The Aβ oligomer means a polymer of Aβ that is a dimer, trimer, tetramer or higher polymer. In terms of the amino acid length of the Aβ used in the present invention, the protein may be constituted by either 40 amino acids (Aβ40) or 42 amino acids (Aβ42). However, in cases where the Aβ is produced from APP having the deletion mutation of glutamic acid at position 693, Aβ40 means an amino acid length of 39, and Aβ42 means an amino acid length of 41. The accumulation of Aβ oligomer means that these Aβ oligomers are aggregated in the cells, and such accumulation can be detected by observing the cells using an antibody against Aβ oligomers (for example, NU1 or 11A1).

In the present invention, examples of the patient who is known to be suffering from Alzheimer's disease include, but are not limited to, patients having a causative gene of familial Alzheimer's disease. Examples of the causative gene include the amyloid precursor protein gene (APP) on chromosome 21, presenilin 1 gene on chromosome 14, mutant-type presenilin 2 gene on chromosome 1, and apolipoprotein E gene ε4 allele on chromosome 19. In the present invention, the somatic cells of a patient who is known to be suffering from Alzheimer's disease may be somatic cells having APP (E693Δ), in which glutamic acid at position 693 of APP is deleted. In the present invention, for example, the APP is the gene of NCBI accession number NM_000484, or the protein encoded by this gene.

In the present invention, instead of the iPS cells prepared from somatic cells of a patient who is known to be suffering from Alzheimer's disease, iPS cells prepared by introduction of APP (E693Δ), in which glutamic acid at position 693 of APP is deleted, may be used. The introduction of the mutant APP can be carried out using the same method as the above-described method of introduction of nuclear reprogramming substances into somatic cells. The introduction of the mutant APP may be carried out for iPS cells, or may be carried out for nerve cells or astrocytes after the differentiation induction described later.

### Method for Differentiation Induction into Nerve Cells

The method of differentiation induction of the above-mentioned iPS cells into neural stem cells is not restricted, and examples of the method which may be used include a differentiation induction method by high-density culture on a fibroblast feeder layer (JP 2008-201792 A), a differentiation induction method by co-culturing with stromal cells (SDIA method) (e.g., WO 2001/088100 or WO 2003/042384) and a differentiation induction method by suspension culture (SFEB method) (WO 2005/123902), and combinations of two or more of these methods.

In another mode for induction of nerve cells, iPS cells induced by the above-described method are separated by an arbitrary method and allowed to form embryoid bodies (EBs), followed by subjecting the cells to adherent culture in an arbitrary medium placed in a coated culture dish. The nerve cells are preferably cerebral cortical neurons.

In the present invention, the nerve cells are cells expressing at least one gene selected from the group consisting of the genes for BF1, βIII tubulin, TuJ1, NeuN, 160 kDa neurofilament protein, MAP2ab, glutamate, synaptophysin, glutamic acid decarboxylase (GAD), tyrosine hydroxylase, GABA, serotonin, TBR1, CTIP2 and SATB2. The nerve cells are preferably cells expressing at least one gene selected from the group consisting of TBR1, CTIP2 and SATB2.

In the present invention, the method of separation may be a mechanical method or use of a separation solution having protease activity and collagenase activity (e.g., Accutase(TM) or Accumax(TM)).

In the separation, the iPS cells are preferably separated into single cells, or may be separated into small clusters or a mixture of small clusters and single cells.

The formation of EBs is usually carried out by suspension culture, but the method of formation of EBs is not limited thereto.

The suspension culture means culturing of cells in a state where the cells are not adhering to the culture dish. Examples of the suspension culture include, but are not limited to, suspension culture performed using a culture dish which is not artificially treated for the purpose of enhancing adhesiveness to cells (for example, by coating treatment with an extracellular matrix or the like), and suspension culture performed using a culture dish which is artificially treated to suppress adhesion (for example, by coating treatment with polyhydroxyethylmethacrylate (poly-HEMA), 2-methacryloyloxyethyl phosphorylcholine (MPC) polymer or Pluronic F-127 (Gibco)). Preferably, a culture dish coated with Pluronic F-127 is used for the suspension culture.

Examples of the agent for coating the culture dish to be used for adherent culture include Matrigel (Becton Dickinson), collagen, gelatin, poly-L-lysine, poly-D-lysine, fibronectin, laminin, heparan sulfate proteoglycan and entactin, and combinations of two or more of these agents. The coating agent is preferably Matrigel.

The culture medium to be used in the present invention may be prepared by adding an additive(s) to a basal medium. The basal medium is not restricted as long as it can be used for culture of animal cells, and examples of the basal medium include Neurobasal medium, Neural Progenitor Basal medium, NS-A medium, BME medium, BGJb medium, CMRL 1066 medium, Glasgow MEM medium, Improved MEM Zinc Option medium, IMDM medium, Medium 199 medium, Eagle MEM medium, αMEM medium, DMEM medium, DMEM/F12 medium, Ham's medium, RPMI 1640 medium and Fischer's medium, and mixtures of two or more of these media. The basal medium is more preferably a mixture of Neurobasal medium and DMEM/F12. Examples of the additive include serum, retinoic acid, Wnt, BMP, bFGF, EGF, HGF, Sonic hedgehog (Shh), brain-derived neurotrophic factor (BDNF), glial cell line-derived neurotrophic factor (GDNF), neurotrophin-3 (NT-3), insulin-like growth factor 1 (IGF1), amino acids, vitamins, interleukins, insulin, transferrin, heparin, heparan sulfate, collagen, fibronectin, progesterone, selenite, B27-supplement (Gibco), B27-supplement (vitamin A-free) (Gibco), N2-supplement (Gibco), ITS-supplement, antibiotics, inhibitors of AMPK and BMP signals (e.g., Dorsomorphin), ALK5 inhibitors (e.g., SB431542) and Knockout Serum Replacement (KSR). Preferred additives are N2-supplement, Dorsomorphin, SB431542, B27-supplement (vitamin A-free), BDNF, GDNF, NT-3 and KSR. The additives employed may be changed in a stepwise manner. For example, the combination of additives may be changed as appropriate from/to: the combination of KSR, Dorsomorphin and SB431542; the combination of N2-supplement, Dorsomorphin and SB431542; and/or the combination of B27-supplement (vitamin A-free), BDNF, GDNF and NT-3.

Examples of a more preferred mode of the medium and the culture conditions include, but are not limited to, conditions where the culture is carried out in the following 3 stages.
(Stage 1) EBs are allowed to form in DMEM/Ham's F12 supplemented with 2 µM dorsomorphin, SB431542 and 5% KSR placed in an MPC polymer-coated culture dish.
(Stage 2) Adhesion culture is performed with DMEM/Ham's F12 supplemented with 2 µM dorsomorphin, SB431542 and N2-supplement placed in a Matrigel-coated culture dish.
(Stage 3) Cells are separated using Accutase, and cultured in neurobasal medium supplemented with B27-supplement (vitamin A-free), 10 ng/ml BDNF, 10 ng/ml GDNF and 10 ng/ml NT-3 placed in a Matrigel-coated culture dish.

The concentration of the iPS cells at the beginning of the culture may be arbitrarily set to allow efficient formation of nerve cells. The concentration of the iPS cells at the beginning of the culture is not restricted, and the concentration is, for example, about 1×10⁴ to about 5×10⁶ cells/ml, preferably about 5×10⁵ to about 2×10⁶ cells/ml.

Other culture conditions such as the culture temperature and the CO₂ concentration may be arbitrarily set. Examples of the culture temperature include, but are not limited to, about 30 to 40°C. The culture temperature is preferably about 37°C. Examples of the CO₂ concentration include, but are not limited to, about 1 to 10%. The CO₂ concentration is preferably about 5%. The O₂ concentration is 1 to 20%. Further, the O₂ concentration may be 1 to 10%.

Examples of the culture period for the differentiation induction include, but are not limited to, 28 days to 84 days. In cases where the differentiation induction is carried out in the above-described stages, the Stage 1 is carried out preferably for 4 days to 12 days, more preferably for 8 days. The Stage 2 is carried out preferably for 8 days to 24 days, more preferably for 16 days. The Stage 3 is carried out preferably for 16 days to 56 days, preferably for 32 days or 48 days.

### Method for Differentiation Induction into Astrocytes

Examples of the method for inducing differentiation of iPS cells into astrocytes in the present invention include a method comprising the steps of: (1) producing neural progenitor cells from iPS cells; (2) culturing the obtained neural progenitor cells in a culture medium supplemented with a neurotrophic factor(s); (3) dissociating the obtained cells; and (4) subjecting the obtained cells to adherent culture in a culture medium supplemented with a neurotrophic factor(s) using an uncoated culture vessel.

In the present invention, the term "neural progenitor cells" means cells that differentiate into neurons or glial cells and express Nestin or NCAM. In the present description, the term "neural progenitor cells" means cells equivalent to neural stem cells, and these two types of cells are not distinguished from each other unless otherwise specified. The term "glial cells" means astrocytes, oligodendrocytes and the like.

In the present invention, the term "astrocytes" means cells that express GFAP or S100β, preferably cells that express GFAP. GFAP is the gene having the sequence of NCBI Accession No. NM_001131019, NM_001242376 or NM_002055.

### <Production of Neural Progenitor Cells>

In the present invention, the differentiation induction of iPS cells into neural progenitor cells may be carried out using a method well known to those skilled in the art, and the method of differentiation induction is not limited. Examples of the method include: (1) a method in which embryoid bodies are formed in a serum-free medium, followed by allowing differentiation (SFEB method) (Watanabe K, et al. Nat Neurosci. 8:288-96, 2005); (2) a method in which ES cells are cultured on stromal cells to cause differentiation (SDIA method) (Kawasaki H, et al. Neuron. 28:31-40, 2000); and (3) a method in which Matrigel supplemented with an agent is used to perform culture (Chambers SM, et al. Nat Biotechnol. 27:275-80, 2009). A preferred method of differentiation induction of iPS cells into neural progenitor cells is a method comprising the step of culturing iPS cells in a culture medium supplemented with a BMP inhibitor and a TGFβ inhibitor.

In a preferred method of differentiation induction of iPS cells into neural progenitor cells in the present invention, iPS cells may be separated by an arbitrary method and then subjected to suspension culture or to adhesion culture using a coated culture vessel. The cells are preferably subjected to suspension culture and then to adherent culture. Examples of the method of separation of human iPS cells herein include a method by mechanical separation, and a separation method using a separation solution having protease activity and collagenase activity (e.g., Accutase (TM) or Accumax (TM)) or a separation solution having only collagenase activity. The method is preferably a method comprising dissociating human pluripotent stem cells using a separation solution having protease activity and collagenase activity (especially preferably Accutase (TM)), and then mechanically and finely dispersing the dissociated cells into single cells. The human iPS cells used in this method are preferably in the form of colonies cultured to 80% confluence with respect to the dish used.

The suspension culture in this step means culturing of cells in a state where the cells are not adhering to the culture vessel. The suspension culture is not limited, and may be carried out using a culture vessel that is not artificially treated for the purpose of enhancing adhesiveness to cells (for example, by coating treatment with an extracellular matrix or the like), or using a culture vessel that is artificially treated such that adhesion is artificially suppressed (for example, by coating treatment with polyhydroxyethylmethacrylate (poly-HEMA) or with a nonionic surfactant polyol (e.g., Pluronic F-127)).

In the adherent culture, the cells are cultured in an arbitrary medium in a coated culture vessel. Examples of the coating agent include Matrigel (BD), collagen, gelatin, laminin, heparan sulfate proteoglycan and entactin, and combinations of two or more of these agents. The coating agent is preferably Matrigel.

The medium in this step may be prepared using, as a basal medium, a medium used for culturing animal cells. Examples of the basal medium include IMDM, Medium 199, Eagle's Minimum Essential Medium (EMEM), αMEM, Dulbecco's modified Eagle's Medium (DMEM), Ham's F12 medium, RPMI 1640 medium, Fischer's medium and Neurobasal Medium (Life Technologies), and mixtures of two or more of these media. The basal medium is preferably DMEM/F12 medium prepared by mixing equal amounts of DMEM and Ham's F12 medium. The medium may contain serum, or may be serum-free. The medium may contain, if necessary, one or more serum replacements such as albumin, transferrin, Knockout Serum Replacement (KSR) (serum replacement for FBS in ES cell culture), N2 supplement (Invitrogen), B27 supplement (Invitrogen), fatty acid, insulin, collagen precursor, trace element, 2-mercaptoethanol and/or 3'-thiolglycerol, and may also contain one or more substances such as lipid, amino acid, L-glutamine, Glutamax (Invitrogen), non-essential amino acid, vitamin, growth factor, low-molecular-weight compound, antibiotic, antioxidant, pyruvic acid, buffer and/or inorganic salt. The medium is preferably DMEM/F12 medium supplemented with KSR, amino acids and L-glutamic acid, or DMEM/F12 medium supplemented with N2 supplement, KSR, amino acids and L-glutamine.

In this process, a BMP inhibitor and a TGFβ inhibitor are preferably added to the medium. The BMP inhibitor, unlike naturally occurring protein-based inhibitors such as Noggin, chordin and follistatin, is a low-molecular-weight inhibitor involved in inhibition of BMP signaling, which mediates binding of BMP (bone morphogenetic protein) to a BMP receptor (type I or type II). This inhibitor should have an action to cause differentiation induction of pluripotent stem cells into neural progenitor cells. Examples of low-molecular-weight BMP inhibitors having such a property include compounds that inhibit BMP2, BMP4, BMP6 or BMP7, which have a capacity to activate a transcription factor SMAD1, SMAD5 or SMAD8, and examples of the compounds include Dorsomorphin (that is, 6-[4-(2-piperidin-1-yl-ethoxy)phenyl]-3-pyridin-4-yl-pyrazolo[1,5-a]pyrimidine) and its derivatives (P. B. Yu et al. (2007), Circulation, 116:II 60; P.B. Yu et al. (2008), Nat. Chem. Biol., 4:33-41; J. Hao et al. (2008), PLoS ONE (www.plozone.org), 3(8):e2904). Dorsomorphin is commercially available, and can be obtained from, for example, Sigma-Aldrich. Dorsomorphin has a biological activity that inhibits the above-described BMP signaling by inhibition of binding of BMP to a BMP receptor. Other examples of the inhibitor include BMP I-type receptor kinase inhibitors such as LDN-193189 (that is, 4-(6-(4-(piperazin-1-yl)phenyl)pyrazolo[1,5-a]pyrimidin-3-yl)quinoline) and its derivatives (Yu PB et al. Nat Med, 14: 1363-9, 2008). LDN-193189 is commercially available, and can be obtained from Stemgent, Inc.

In cases where the BMP inhibitor is Dorsomorphin, examples of its concentration in the medium include 0.1 mM, 0.2 mM, 0.3 mM, 0.4 mM, 0.5 mM, 0.6 mM, 0.7 mM, 0.8 mM, 0.9 mM, 1 mM, 2 mM, 3 mM, 4 mM, 5 mM, 10 mM, 20 mM, 30 mM, 40 mM, 50 mM and 100 mM. The concentration is preferably 2 mM.

The TGFβ inhibitor is a low-molecular-weight inhibitor that interferes with signaling by the TGFβ family, and examples of the TGFβ inhibitor include SB431542 and SB202190 (these are described in R. K. Lindemann et al., Mol. Cancer 2:20(2003)), SB505124 (GlaxoSmithKline), NPC30345, SD093, SD908, SD208 (Scios), LY2109761, LY364947 and LY580276 (Lilly Research Laboratories). SB431542 is preferred.

In cases where the TGFβ inhibitor is SB431542, examples of its concentration in the medium include 1 mM, 2 mM, 3 mM, 4 mM, 5 mM, 6 mM, 7 mM, 8 mM, 9 mM, 10 mM, 20 mM, 30 mM, 40 mM, 50 mM, 60 mM, 70 mM, 80 mM, 90 mM and 100 mM. The concentration is preferably 10 mM.

The culture temperature is about 30 to 40°C, preferably about 37°C, although the culture temperature is not limited. The culture is carried out in an air atmosphere containing CO₂. The CO₂ concentration is preferably about 2 to 5%, preferably 5%. The culture period is at least 20 days, and examples of the culture period include 21 days, 24 days, 27 days, 30 days, 33 days, 36 days, 39 days and 42 days. The culture period is preferably 24 days.

### <Step of Culturing Neural Progenitor Cells in Culture Medium Supplemented with Neurotrophic Factor(s)>

The culture medium used in the step of culturing the neural progenitor cells of the present invention may be prepared using, as a basal medium, a medium for culturing animal cells. Examples of the basal medium include IMDM, Medium 199, Eagle's Minimum Essential Medium (EMEM), αMEM, Dulbecco's modified Eagle's Medium (DMEM), Ham's F12 medium, RPMI 1640 medium, Fischer's medium and Neurobasal Medium (Life Technologies), and mixtures of two or more of these media. The basal medium is preferably Neurobasal Medium. The medium may contain serum, or may be serum-free. The medium may contain, if necessary, one or more serum replacements such as albumin, transferrin, Knockout Serum Replacement (KSR) (serum replacement for FBS in ES cell culture), N2 supplement (Invitrogen), B27 supplement (Invitrogen), fatty acid, insulin, collagen precursor, trace element, 2-mercaptoethanol and/or 3'-thiolglycerol, and may also contain one or more substances such as lipid, amino acid, L-glutamine, Glutamax (Invitrogen), non-essential amino acid, vitamin, growth factor, low-molecular-weight compound, antibiotic, antioxidant, pyruvic acid, buffer and/or inorganic salt. A preferred medium is Neurobasal Medium supplemented with B27 supplement and Glutamax.

In the present invention, the culture medium used for the step of culturing neural progenitor cells preferably contains a neurotrophic factor. The neurotrophic factor herein means a ligand for a membrane receptor playing an important role in survival and maintenance of functions of motor neurons, and examples of the neurotrophic factor include Nerve Growth Factor (NGF), Brain-derived Neurotrophic Factor (BDNF), Neurotrophin 3 (NT-3), Neurotrophin 4/5 (NT-4/5), Neurotrophin 6 (NT-6), basic FGF, acidic FGF, FGF-5, Epidermal Growth Factor (EGF), Hepatocyte Growth Factor (HGF), Insulin, Insulin Like Growth Factor 1 (IGF 1), Insulin Like Growth Factor 2 (IGF 2), Glia cell line-derived Neurotrophic Factor (GDNF), TGF-b2, TGF-b3, Interleukin 6 (IL-6), Ciliary Neurotrophic Factor (CNTF) and LIF. The neurotrophic factor(s) preferred in the present invention is/are a factor(s) selected from the group consisting of GDNF, BDNF and NT-3.

In the step of culturing neural progenitor cells, the culture may be carried out using a coated culture vessel. Examples of the coating agent include Matrigel (BD), collagen, gelatin, laminin, heparan sulfate proteoglycan and entactin, and combinations of one or more of these agents. The coating agent is preferably Matrigel.

In terms of the culture conditions, the culture temperature is about 30 to 40°C, preferably about 37°C, although the culture temperature is not limited. The culture is carried out in an air atmosphere containing CO₂, and the CO₂ concentration is preferably about 2 to 5%.

The culture period is not limited since long-term culture does not cause a problem, and examples of the culture period include not less than 20 days, not less than 30 days, not less than 40 days, not less than 50 days, not less than 60 days, not less than 70 days, not less than 80 days, not less than 90 days, and periods longer than these. The culture period is preferably not less than 66 days.

The concentration of the above-described neurotrophic factor to be added may be arbitrarily selected by those skilled in the art in consideration of the effect of the factor, and examples of the concentration include 1 ng/ml, 2 ng/ml, 3 ng/ml, 4 ng/ml, 5 ng/ml, 6 ng/ml, 7 ng/ml, 8 ng/ml, 9 ng/ml, 10 ng/ml, 15 ng/ml, 20 ng/ml, 30 ng/ml, 40 ng/ml, 50 ng/ml, 60 ng/ml, 70 ng/ml, 80 ng/ml, 90 ng/ml and 100 ng/ml. The concentration is preferably 10 ng/ml.

### <Step of Dissociating Cells>

In the step of dissociating the cells, cells adhering to each other and forming a population are dissociated (separated) into individual cells. Examples of the method for dissociating the cells include a method in which the cells are mechanically dissociated, and a method in which a dissociation solution having protease activity and collagenase activity (e.g., Accutase (TM) or Accumax (TM)) or a dissociation solution having only collagenase activity is used. The method is preferably a method in which a dissociation solution having protease activity and collagenase activity (especially preferably Accutase (TM)) is used to dissociate the cells.

### <Step of Subjecting Dissociated Cells to Adherent Culture in Culture Medium Supplemented with Neurotrophic Factor(s) Using Uncoated Culture Vessel>

The uncoated culture vessel means a dish, plate or flask for cell culture that is widely used by those skilled in the art. The vessel has an arbitrary shape and has not been subjected to a treatment process using a coating agent before use in the culture. The vessel is preferably a polystyrene culture vessel. Examples of the coating agent include Matrigel (BD), collagen, gelatin, laminin, heparan sulfate proteoglycan and entactin, and, in this step, it is preferred to use a culture vessel that has not been treated at least with these coating agents.

In the culture after dissociation of cells, the same culture medium supplemented with a neurotrophic factor(s) as described above can be used. The culture period is not limited since long-term culture does not cause a problem, and examples of the culture period include not less than 5 days, not less than 10 days, not less than 15 days, not less than 20 days, not less than 25 days, not less than 30 days, not less than 35 days, not less than 40 days, not less than 45 days, not less than 50 days, and periods longer than these. The culture period is preferably not less than 30 days.

### <Additional Step>

In the present invention, the production of astrocytes may be carried out by further dissociating the obtained cells and subjecting the dissociated cells to adherent culture using an uncoated culture vessel, in a culture medium that does not contain a factor selected from the group consisting of GDNF, BDNF and NT-3. The dissociation of cells can be carried out by the same method as described above, and the dissociation is preferably carried out using a dissociation solution having protease activity and collagenase activity.

In the present invention, the culture medium that does not contain a factor selected from the group consisting of GDNF, BDNF and NT-3 can be prepared using, as a basal medium, a medium used for culturing animal cells. Examples of the basal medium include IMDM, Medium 199, Eagle's Minimum Essential Medium (EMEM), αMEM, Dulbecco's modified Eagle's Medium (DMEM), Ham's F12 medium, RPMI 1640 medium, Fischer's medium and Neurobasal Medium (Life Technologies), and mixtures of two or more of these media. The medium is preferably Neurobasal Medium. The medium may contain serum, or may be serum-free. The medium may contain, if necessary, one or more serum replacements such as albumin, transferrin, Knockout Serum Replacement (KSR) (serum replacement for FBS in ES cell culture), N2 supplement (Invitrogen), B27 supplement (Invitrogen), fatty acid, insulin, collagen precursor, trace element, 2-mercaptoethanol and/or 3'-thiolglycerol, and may also contain one or more substances such as lipid, amino acid, L-glutamine, Glutamax (Invitrogen), non-essential amino acid, vitamin, growth factor, low-molecular-weight compound, antibiotic, antioxidant, pyruvic acid, buffer and/or inorganic salt. DMEM/F12 supplemented with N2 supplement and Glutamax, and DMEM/F12 supplemented with serum and Glutamax are preferred as the medium that does not contain a factor selected from the group consisting of GDNF, BDNF and NT-3.

The period of this step is not limited since long-term culture does not cause a problem, and examples of the culture period include not less than 5 days, not less than 10 days, not less than 15 days, not less than 20 days, not less than 25 days, not less than 30 days, not less than 35 days, not less than 40 days, not less than 45 days, not less than 50 days, and periods longer than these. The period is preferably not less than 20 days or not less than 30 days.

In terms of the culture conditions in this step, the culture temperature is about 30 to 40°C, preferably about 37°C, although the culture temperature is not limited. The culture is carried out in an air atmosphere containing CO₂, and the CO₂ concentration is preferably about 2 to 5%.

The step of dissociation and culture of the cells is preferably carried out at least once for increasing the efficiency of obtaining astrocytes. Examples of the number of times of the step include not less than 2, not less than 3, not less than 4 and not less than 5. The number of times of the step is more preferably 3.

### <Method for Selecting Astrocytes>

After the step of culturing neural progenitor cells in a culture medium supplemented with a neurotrophic factor(s), neurons, in addition to astrocytes, may be produced at the same time. However, since astrocytes are more likely to adhere to uncoated culture vessels than neurons, use of this method allows selective acquisition of astrocytes at high efficiency from a group of cells containing both astrocytes and neurons. More specifically, the method comprises the above-described step of separating the cells and step of culturing the cells using an uncoated culture vessel.

### Method for Screening Prophylactic and/or Therapeutic Agent for Alzheimer's Disease

The present invention provides a method for screening of a candidate substance for a prophylactic and/or therapeutic agent for Alzheimer's disease by bringing a test substance into contact with iPS cell-derived neural cells (nerve cells or astrocytes) obtained as described above, and using various indices. The iPS cells preferably used in the present invention are iPS cells derived from a patient with Alzheimer's disease, or iPS cells in which an exogenous mutant APP has been introduced. The mutation of APP or mutant APP means an iPS cell having deletion mutation of glutamic acid at position 693 of APP.

In another mode, the iPS cell used in the present invention is preferably an iPS cell from which a neural cell showing accumulation of Aβ oligomers can be prepared. In the present invention, examples of such an iPS cell include an iPS cell derived from a patient with sporadic Alzheimer's disease, iPS cell having a mutation in the endogenous APP, and iPS cell in which an exogenous mutant APP has been introduced.

In one mode of the present invention in which the amount of Aβ is used as an index, screening of a therapeutic and/or prophylactic agent for Alzheimer's disease is possible by a method comprising the steps of:
(a) bringing a candidate substance into contact with neural cells (nerve cells or astrocytes) derived from iPS cells;
(b) measuring the amount of Aβ oligomers in the nerve cells; and
(c) selecting the candidate substance as a therapeutic and/or prophylactic agent for Alzheimer's disease if the amount is decreased as compared to a case where the candidate substance is not brought into contact.

Examples of the method for measuring the amount of Aβ oligomers in nerve cells include a method in which the obtained cells are washed and a cell lysate is obtained using an arbitrary lysing buffer, which cell lysate is then used for the measurement. In such a case, an immunoassay may be used for the measurement. Examples of the immunoassay include, but are not limited to, ELISA, Western blotting, immunoprecipitation, slot or dot blot assay, immunohistostaining, radioimmunoassay (RIA), fluoroimmunoassay, and immunoassay using the avidin-biotin or streptavidin-biotin system. The immunoassay is preferably ELISA such as sandwich ELISA. Another example of the method for measuring Aβ oligomers in nerve cells is a method in which the obtained cells are subjected to immunohistostaining using an antibody against Aβ oligomers and then to measurement of the stained area using a cell imaging device. Examples of the cell imaging device include the IN Cell Analyzer.

In the screening method of the present invention, an arbitrary test substance may be used as the candidate substance. The test substance may be any known compound or novel compound, and examples of the test substance include cell extracts, cell culture supernatants, microbial fermentation products, extracts derived from marine organisms, plant extracts, purified proteins and crude proteins, peptides, nonpeptide compounds, synthetic low molecular compounds and naturally occurring compounds. In the present invention, the test substance may be obtained by using any of a number of approaches in combinatorial library methods known in the art, such as (1) the biological library method, (2) the synthetic library method using deconvolution, (3) the "one-bead one-compound" library method and (4) the synthetic library method using affinity chromatography selection. Application of the biological library method using affinity chromatography selection is limited to peptide libraries, but the other 4 types of approaches can be applied to low-molecular compound libraries of peptides, nonpeptide oligomers or compounds (Lam (1997) Anticancer Drug Des. 12: 145-67). Examples of synthetic methods of molecular libraries can be found in the art (DeWitt et al. (1993) Proc. Natl. Acad. Sci. USA 90: 6909-13; Erb et al. (1994) Proc. Natl. Acad. Sci. USA 91: 11422-6; Zuckermann et al. (1994) J. Med. Chem. 37: 2678-85; Cho et al. (1993) Science 261: 1303-5; Carell et al. (1994) Angew. Chem. Int. Ed. Engl. 33: 2059; Carell et al. (1994) Angew. Chem. Int. Ed. Engl. 33: 2061; Gallop et al. (1994) J. Med. Chem. 37: 1233-51). The compound libraries may be prepared as solutions (see Houghten (1992) Bio/Techniques 13: 412-21) or beads (Lam (1991) Nature 354: 82-4), chips (Fodor (1993) Nature 364: 555-6), bacteria (US 5,223,409 B), spores (US 5,571,698 B, US 5,403,484 B and US 5,223,409 B), plasmids (Cull et al. (1992) Proc. Natl. Acad. Sci. USA 89: 1865-9) or phages (Scott and Smith (1990) Science 249: 386-90; Devlin (1990) Science 249: 404-6; Cwirla et al. (1990) Proc. Natl. Acad. Sci. USA 87: 6378-82; Felici (1991) J. Mol. Biol. 222: 301-10; US 2002103360 B).

In one mode of the screening method, the amount of Aβ oligomers in neural cells that were not brought into contact with the test substance is compared with the amount of Aβ oligomers in neural cells that were brought into contact with the test substance, and in cases where the amount of Aβ oligomers is lower in the cells that were brought into contact, the test substance is selected as a candidate substance for a prophylactic and/or therapeutic agent for Alzheimer's disease.

In another mode of the present invention in which at least one index selected from the group consisting of the ER stress level, caspase 4 activity, transgelin level and oxidative stress level is used, screening of a therapeutic and/or prophylactic agent for Alzheimer's disease is possible by a method comprising the steps of:
(a) bringing a candidate substance into contact with neural cells (nerve cells or astrocytes) derived from iPS cells;
(b) measuring at least one index selected from the group consisting of the ER stress level, caspase 4 activity, transgelin level and oxidative stress level in the nerve cells; and
(c) selecting the candidate substance as a therapeutic and/or prophylactic agent for Alzheimer's disease if the level(s) is/are decreased as compared to a case where the candidate substance is not brought into contact.

In the present invention, the endoplasmic reticulum (ER) stress means an adverse effect caused by accumulation of a denatured protein which does not have a normal folded structure, in endoplasmic reticulum. The ER stress level can be measured by measuring the level of endoplasmic reticulum stress response. The endoplasmic reticulum stress response herein means a decrease in expression of a causative protein of the denatured protein, an increase in a molecular chaperone, or the like. Measurement of the ER stress level is therefore carried out by measurement of the level of an ER stress marker such as a molecular chaperone. Examples of the ER stress marker herein include, but are not limited to, BiP (Binding Immunoglobulin Protein), XBP-1 (X Box-binding Protein 1), eIF2A (Eukaryotic translation Initiation Factor 2 A), XIAP (X-chromosome-linked Inhibitor of Apoptosis), ATF4 (Activating Transcription Factor 4), IRE1 (Inositol-Requiring Enzyme 1), PERK (PKR-Like ER Kinase) and ATF6 (Activating Transcription Factor 6). The ER stress marker is preferably BiP.

The ER stress level can be measured by, for example, using an immunoassay. Examples of the immunoassay include, but are not limited to, ELISA, Western blotting, immunoprecipitation, slot or dot blot assay, immunohistostaining, radioimmunoassay (RIA), fluoroimmunoassay, and immunoassay using the avidin-biotin or streptavidin-biotin system. The immunoassay is preferably ELISA such as sandwich ELISA.

Another examples of the method for measuring the ER stress level is a method in which the obtained cells are subjected to immunohistostaining using an antibody against an ER stress marker and then to measurement of the stained area using a cell imaging device. Examples of the cell imaging device include IN Cell Analyzer.

The test substance obtained by such screening can be used as a prophylactic and/or therapeutic agent for Alzheimer's disease.

In the present invention, the caspase 4 activity can be quantified by measuring the level of activated caspase 4. The activated caspase 4 herein is in a cleaved state and has cysteine protease activity. The activated caspase 4 can be measured by an immunoassay using an antibody specific to the cleaved site. Alternatively, the activity can be measured using a commercially available fluorescent caspase substrate.

In the present invention, transgelin is the gene represented by NCBI Accession NO. NM_001001522 or NM_003186, and its level can be recognized as the mRNA or protein level. The level may be a level recognized using only a part of the gene. In cases of the mRNA, the level may be recognized with a continuous sequence having a length of at least 15 bases in the ORF sequence, or with the complementary strand thereof. In cases of the protein, the level may be recognized with a continuous sequence having a length of at least 3 to 6 amino acids.

In cases where the transgelin level in the present invention is measured as the mRNA level, the measurement may be carried out by a known method that enables specific recognition and detection of a gene, mRNA or cDNA, such as Northern blotting, Southern blotting, quantitative RT-PCR, real-time PCR or *in situ* hybridization according to a conventional procedure using a primer or probe. In cases where the transgelin level is measured as the protein level, the measurement may be carried out by an immunoassay.

In the present invention, the oxidative stress means a phenomenon in which protein, lipid or DNA is damaged by reactive oxygen species (ROS) or reactive nitrogen species (RNS). The oxidative stress level can be quantified by measuring the level of an oxidatively modified substrate, the level of an oxidative-stress-eliminating enzyme whose expression is increased, or the level of RNS or ROS. Examples of the oxidative modification herein include phosphorylation, hydroxylation, nitration and carbonylation. The oxidative-stress-eliminating enzyme is an enzyme having a function to protect cells against oxidative stress, and examples of the enzyme include proteins having oxidoreductase, peroxidase or peroxiredoxin activity. Measurement of the oxidative stress can be carried out by measuring the level of DNA cleavage, 8-hydroxyguanosine, malondialdehyde, 4-hydroxynonenal, 8-isoprostane, PRDX1 (NCBI Accession NO. NM_002574), PRDX4 (NCBI Accession NO.NM_006406), PRDX5 (NCBI Accession NO. NM_012094), PXDN (NCBI Accession NO.NM_012293), MGST3 (NCBI Accession NO. NM_004528), MED31 (NCBI Accession NO.NM_016060), RNS (e.g., nitrogen monoxide), ROS (e.g., hydrogen peroxide, superoxide anion radical or hydroxy radical) or the like using a method well known to those skilled in the art.

In another mode of the present invention in which the number of nerve cells derived from iPS cells is used as an index, screening of a therapeutic and/or prophylactic agent for Alzheimer's disease can be carried out by a method comprising the steps of:
(a) bringing a candidate substance into contact with nerve cells derived from iPS cells;
(b) measuring the viable cell number of the nerve cells or an alternative value thereof; and
(c) selecting the candidate substance as a therapeutic and/or prophylactic agent for Alzheimer's disease if the viable cell number or alternative value thereof is increased as compared to a case where the candidate substance is not brought into contact.

In the present invention, in order to clearly detect a decrease in the number of nerve cells, the nerve cells may be cultured in a medium containing no neurotrophic factor before measurement of the number of nerve cells. In another mode, the cells may be cultured in a medium supplemented with hydrogen peroxide, before measurement of the number of nerve cells. The concentration of the hydrogen peroxide used in this mode may be, for example, from 50 µM to 500 µM, preferably from 100 µM to 400 µM, more preferably 200 µM.

In the present invention, in order to count only nerve cells, the nerve cells may be specifically stained before the counting. The staining may be carried out for at least one gene selected from the group consisting of BF1, βIII tubulin, TuJ1, NeuN, 160 kDa neurofilament protein, MAP2ab, glutamate, synaptophysin, glutamic acid decarboxylase (GAD), tyrosine hydroxylase, GABA, serotonin, Synapsin I, TBR1, CTIP2 and SATB2. Alternatively, the staining may be carried out for a marker gene(s) induced by the promoter(s) responsible for expression of the above gene(s), instead of the above gene(s) itself/themselves. Examples of the marker gene include genes encoding fluorescent proteins such as GFP, RFP and YFP.

In the present invention, the method for measuring the viable cell number may be counting of the nerve cells obtained in the step (a) by visual observation using a microscope or the like, and the viable cells may be stained prior to the counting, by a method well known to those skilled in the art such as use of MTT. Alternatively, the counting may be carried out using an automated cell counting device or the like. Alternatively, the reciprocal of the number of dead cells counted by measuring LDH or using trypan blue may be employed as an alternative value of the viable cell number.

### Kit for Screening of Prophylactic and/or Therapeutic Agent for Alzheimer's Disease

The kit for screening of a prophylactic and/or therapeutic agent for Alzheimer's disease of the present invention contains:
(a) a nerve cell and/or astrocyte derived from an iPS cell having a mutant-type APP; and/or
(b) a reagent for measuring at least one index selected from the group consisting of Aβ oligomers, ADAM17, BACE1, BiP, cleaved caspase 4, PRDX4 and reactive oxygen species.

In another mode, the kit for screening of a prophylactic and/or therapeutic agent for Alzheimer's disease of the present invention comprises: (a) an iPS cell having a mutant-type APP; (b) a reagent for inducing differentiation into neural cells (nerve cells or astrocytes); and (c) a reagent(s) for measuring the amount of Aβ, the ER stress level, the amount of transgelin, and/or the number of nerve cells.

In another mode, the kit for screening of a prophylactic and/or therapeutic agent for Alzheimer's disease of the present invention comprises: (a) a somatic cell having a mutant-type APP; (b) a reprogramming substance for iPS cell production; (c) a reagent for inducing differentiation into neural cells (nerve cells or astrocytes); and (d) a reagent(s) for measuring the amount of Aβ, the ER stress level, the amount of transgelin, and/or the number of nerve cells.

In the present invention, the mutation of APP is a mutation that may cause Alzheimer's disease, and examples of the mutation include the E693Δ mutation.

As reprogramming substances for production of iPS cells, the reprogramming substances in the above-mentioned production method for iPS cells may be used. To enhance the induction efficiency of iPS cells upon the nuclear reprogramming, other factors may also be included. At least one factor selected from the group consisting of the OCT family, MYC family, KLF family and SOX family is preferably contained.

As reagents for inducing differentiation into nerve cells or astrocytes, the reagents described above for the differentiation induction method may be used.

The reagent for measuring the amount of Aβ oligomers, BiP, cleaved caspase 4, PRDX4 or ROS may be any substance as long as the substance can recognize the index used in each measurement method. In cases where a protein is to be measured, examples of the reagent include specific antibodies, and, in cases of ROS, examples of the reagent include fluorescent probes that capture reactive oxygen species.

The kit for screening of a prophylactic and/or therapeutic agent for Alzheimer's disease may also contain the above-described arbitrary test substance.

The kit for screening of a prophylactic and/or therapeutic agent for Alzheimer's disease may further contain a document and/or instruction that describe(s) a procedure for production of iPS cells and/or a procedure for differentiation induction.

### EXAMPLES

The present invention is described below in more detain by way of Examples, but the scope of the present invention is not limited to the Examples.

### Example 1

### Establishment of iPS cells (iPSCs)

Dermal fibroblasts (HDFs) were prepared from 3-mm explants obtained, with patient consent, by skin biopsy from a patient with Alzheimer's disease having the E698 deletion mutation (E693Δ) in APP, a patient with Alzheimer's disease having a substitution mutation from valine to leucine at position 717 (V717L), and two patients with sporadic Alzheimer's disease. One to two weeks later, fibroblasts grown from the explants were subcultured. Subsequently, using an episomal vector, human cDNAs (SOX2, KLF4, OCT4, L-MYC and LIN28) as reprogramming factors, and p53 shRNA were introduced to the HDFs (Okita et al., Nat Methods. May; 8(5):409-12.2011). Several days after the introduction, the fibroblasts were recovered, and plated again onto an SNL feeder cell layer. On the next day, the medium was replaced with a medium for primate embryonic stem cells (Reprocell, Kanagawa, Japan) supplemented with 4 ng/ml bFGF (Wako Chemicals, Osaka, Japan). The medium was replaced every other day. Thirty days after the introduction, colonies of iPS cells were picked up (these colonies are referred to as AD(E693Δ)-1, AD(E693Δ)-2, AD(E693Δ)-3, AD(V717L)-1, AD(V717L)-2, AD(V717L)-3, AD(sporadic)-1 and AD(sporadic)-2, respectively).

Three control iPSC lines having no mutation in the APP gene were used in the present invention. One of these lines was iPS cells prepared earlier (Control-3 (409B2)) (Okita et al., Nat Methods. May;8(5):409-12.201 1), and the other two were prepared by the same method as described above from non-AD patients with patient consent (Control-1 and -2). The properties of these iPS cells were investigated by the methods described below.

### Analysis by Immunocytochemistry

The cells were fixed in 4% paraformaldehyde (pH 7.4) at room temperature for 30 minutes, and then washed with PBS. Thereafter, the cells were permeabilized in PBS supplemented with 0.2% Triton X-100 at room temperature for 10 minutes, and then washed with PBS. Non-specific binding was blocked using PBS supplemented with 10% donkey serum at room temperature for 60 minutes. Using a primary antibody, the cells were incubated at 4°C overnight, and then labeled with an appropriate fluorescently tagged secondary antibody. For labeling the nuclei, DAPI (Life Technologies) was used. Fluorescence images were obtained with an FV1000 confocal laser microscope (OLYMPUS, Tokyo, Japan), LSM710 microscope (Carl Zeiss, Gottingen, Germany) or Delta Vision (Applied Precision, Issaquah, WA). In this process, an anti-NANOG antibody (1:10; R&D Systems) was used as the primary antibody. As a result, expression of endogenous pluripotency markers could be confirmed for all cases of iPSCs derived from the controls and AD patients (Figs. 1A and 2A).

### Karyotype Analysis and Genotype Analysis

Karyotype analysis was carried out by a method described earlier. As a result, all iPSCs showed the normal karyotype. Genotype analysis on APP single nucleotide mutations was carried out by direct determination of the base sequence by PCR amplification of genomic DNA (3100 Genetic Analyzer, Applied Biosystems, Life Technologies, CA). As a result, the two types of iPSCs derived from AD patients were confirmed to have mutations (E693Δ and V717L) in APP (Figs. 1B and 2B).

### Teratoma Formation Assay

Undifferentiated iPSCs were recovered by dissociation using the CTK solution, and the precipitate was suspended in DMEM/F12. The cells were subcutaneously transplanted to NOG mice (Central Institute for Experimental Animals, Kawasaki, Japan). Eight weeks after the transplantation, the tumor was cut out, and fixed in PBS containing 4% formaldehyde. The paraffin-embedded tissue was sectioned and stained with hematoxylin and eosin. As a result, all types of iPSCs used in the present Example showed differentiation into tridermoma (teratoma), and were similar to each other to the extent that they could not be distinguished from each other in terms of pluripotency (part of the results are shown in Fig. 1C).

### In Vitro Differentiation

The induced control iPSCs and AD-iPSCs were dissociated using the CTK solution, and recovered. The obtained cell clusters were transferred onto petri dishes containing DMEM/F12 supplemented with 20% knockout serum replacement (KSR, Life Technologies), 2 mM L-glutamine, 0.1 M non-essential amino acids, 0.1 M 2-mercaptoethanol (Life Technologies) and 0.5% penicillin/streptomycin to allow formation of embryoid bodies (EBs). During this, the medium was replaced every other day. After 8 days of the culture, in order to promote differentiation, the cells were plated on a gelatin-coated cover glass, and then cultured in DMEM + 10% fetal bovine serum for additional 8 days. As a result, all types of iPSCs used in the present Example showed differentiation into tridermoma, and were similar to each other to the extent that they could not be distinguished from each other in terms of pluripotency (part of the results are shown in Fig. 1D).

### Bisulfite Genomic Sequencing

For bisulfite modification, genomic DNA (1 µg) from the iPSCs was treated using EZ DNA methylation Gold Kit (Zymoresearch, Irvine, CA). Subsequently, a CpG region conserved in the Oct-4 promoter and a CpG region conserved in the Nanog promoter were amplified by PCR using ExTaq Hot start version (TaKaRa BIO, Shiga, Japan). Each of the obtained PCR products were subcloned into the pCR4 vector (Life Technologies), and the base sequences of 10 clones from each product were confirmed by sequencing using the Sp6 universal primer. As a result, the iPSCs used in the present Example were confirmed to be highly demethylated in each of the regions of Oct-4 and Nanog promoters assayed.

### Example 2

### Differentiation Induction into Cerebral Cortical Neurons (Nerve Cells)

In order to obtain iPS cell-derived cerebral cortical neurons, a method reported earlier (Nat Biotechnol. 2009;27:275-280 and PLoS One. 2009;4:e6722) was used with modification. Briefly, the method was as follows. iPSCs obtained by the above-described method were dissociated into single cells, and then allowed to cause reaggregation in 5% DFK medium (DMEM/Ham's F12 (Gibco), 5% KSR (Gibco), NEAA (Invitrogen), L-glutamine (Sigma-Aldrich), 0.1 M 2-mercaptoethanol (Invitrogen)) supplemented with 2 µM dorsomorphin and SB431542 placed in a U-bottom 96-well plate (Greiner bio-one) coated with Pluronic F-127 (Sigma-Aldrich), to allow formation of embryoid bodies (EBs) (the neural induction stage (P1): from Day 0 to Day 8).

Subsequently, the obtained EBs were transferred to a 6-well plate coated with Matrigel (Becton Dickinson), and cultured in DF medium (DMEM/Ham's F12, NEAA, L-glutamine, 0.1 M 2-mercaptoethanol) supplemented with 2 µM dorsomorphin, SB431542 and N2 supplement (Invitrogen) (the patterning stage (P2): Day 8 to Day 24). As a result, a number of neural progenitor cells (nestin-positive cells) could be observed. Thereafter, the cells were separated using Accutase (Innovative Cell Technologies, Inc.), and transferred to a Matrigel-coated 24-well plate, followed by culturing the cells in the neurobasal (Gibco) medium supplemented with B27 (vitamin A-free) (Gibco), 10 ng/ml BDNF, 10 ng/ml GDNF and 10 ng/ml NT-3 (the neural maturation stage (P3): Day 24 to Day 56, or Day 24 to Day 72).

As a result of 56 days of the culture, expression of cerebral cortical neuron subtype markers TBR1, CTIP2 and SATB2 was found in cerebral cortical neurons (which may be hereinafter referred to as nerve cells) whose differentiation was induced from iPSCs derived from the control or AD(E693Δ) patient. Thus, differentiation of these iPSCs into cerebral cortical neurons could be confirmed (Figs. 1E and 1F).

Similarly, as a result of 72 days of the culture, expression of TUJ1, TBR1 and SATB2 was found in nerve cells whose differentiation was induced from iPSCs derived from the control, AD(E693Δ) patient, AD(V717L) patient or sporadic-AD patient, and differentiation of these iPSCs into cerebral cortical neurons to the same extent could be confirmed (Fig. 2C).

### Example 3

### Differentiation Induction into Astrocytes

### (1) Induction into Neural Progenitor Cells

iPSCs obtained by the above method were dissociated using Accutase (Innovative Cell Technologies). The dissociated iPSCs were suspended in DFK 5% medium (DMEM/Ham's F12 (Gibco) supplemented with 5% KSR (Invitrogen), L-glutamine (Sigma-Aldrich) and 0.1 M 2-mercaptoethanol (Invitrogen)) supplemented with 2 µM Dorsomorphin (Sigma-Aldrich) and 10 µM SB431542 (Cayman Chemical), and then plated in a U-bottom 96-well plate coated with 2% Pluronic F-127 (Sigma-Aldrich) solution in ethanol, to allow formation of embryoid bodies (EBs). This was followed by 8 days of suspension culture. Subsequently, the obtained EBs were transferred to a 6-well plate coated with Matrigel (BD), and cultured for 16 days by adherent culture in DFK 5% medium supplemented with 1×N2 supplement (Invitrogen), 2 µM Dorsomorphin and 10 µM SB431542 (24 days of culture in total), to obtain neural progenitor cells.

### (2) Induction into Astrocytes

The obtained neural progenitor cells were dissociated using Accutase (Innovative Cell Technologies), and cultured for 66 days by adherent culture in Neurobasal medium (Invitrogen) supplemented with 1×B27 without Vitamin A (Invitrogen), 1×Glutamax (Invitrogen), 10 ng/ml BDNF, 10 ng/ml GDNF and 10 ng/ml NT-3 using a Matrigel-coated 12-well plate (90 days of culture in total). Subsequently, the obtained cells were dissociated using Accutase and transferred to an uncoated 6-cm dish, followed by performing adherent culture for 30 days in Neurobasal medium supplemented with 1×B27 without Vitamin A, 1×Glutamax, 10 ng/ml BDNF, 10 ng/ml GDNF and 10 ng/ml NT-3 (120 days of culture in total). The cells without adhesion at this time died by anoikis. The adhered cells were dissociated using Accutase, and transferred to an uncoated 6-cm dish, followed by performing adherent culture for 30 days in DMEM/F12, Glutamax (Invitrogen) supplemented with 1×N2 supplement (150 days of culture in total). Further, the cells obtained twice were dissociated, and cultured for 30 days under the same conditions, to obtain GFAP-positive astrocytes (200 days of culture in total).

### Example 4

### Aβ Secretion from Nerve Cells Derived from AD-iPSCs

In order to test the hypothesis that extracellular Aβ could be reduced in nerve cells derived from AD-iPSCs, the amounts of extracellular Aβ40 and Aβ42 in nerve cells and astrocytes were analyzed. The amounts of extracellular Aβ40 and Aβ42 were measured as described earlier (Yahata et al., PLoS One. 6(9):e25788. 2011), by recovering the culture supernatant after 2 days of culture and then subjecting the sample supernatant to sandwich ELISA (Wako) using the combination of a monoclonal antibody specific to the middle part of Aβ and a monoclonal antibody specific to the C-terminus of Aβ40 or Aβ42. As a result, the amounts of both Aβ40 and Aβ42 were found to be significantly decreased in nerve cells and astrocytes derived from AD(E693Δ)-iPSCs (Figs. 3A and 3B). In terms of the calculated value of Aβ42/Aβ40, the nerve cells did not show any difference, but the astrocytes showed a lower value in the nerve cells derived from AD(E693Δ)-iPSCs. On the other hand, significantly higher secretion of Aβ42 was found in the nerve cells derived from AD(V717L)-iPSCs. To these nerve cells, 1 µM β-Secretase inhibitor IV (BSI) was added, and changes in the amount of Aβ secreted were investigated. As a result, the nerve cells derived from AD(E693Δ)-iPSCs showed no change in the amounts of Aβ40 and Aβ42 secreted, but the nerve cells derived from the control iPSCs, AD(V717L)-iPSCs and sporadic AD-iPSCs showed decreases in the amounts of Aβ40 and Aβ42 secreted.

### Example 5

### Accumulation of Aβ Oligomers in Nerve Cells and Astrocytes Derived from AD-iPS Cells

In order to investigate whether nerve cells and astrocytes derived from AD-iPSCs have Aβ oligomers therein, an Aβ oligomer-specific antibody NU-1 (Gong Y et al., Proc Natl Acad Sci U S A. 100:10417-22.2003) or 11A1 was used to perform immunocytochemical analysis of iPSC-derived nerve cells and astrocytes. As a result, spots of Aβ oligomers could be observed, and they were found to be remarkably increased in nerve cells derived from AD(E693Δ)-iPSCs (Fig. 4A). These spot structures could be seen also in astrocytes derived from AD-iPSCs, but could be hardly seen in the fibroblasts from which the cells were derived. Quantitative analysis of Aβ oligomers in MAP2-positive cells was performed by specifically staining the oligomers. As a result, it was found that Aβ oligomer-positive spot structures were significantly increased in a culture of nerve cells derived from AD-iPSCs as compared to that of control nerve cells (Fig. 4B). Further, use of 11A1, which is another antibody against Aβ, also gave a similar result. A dot blot analysis that was subsequently carried out also showed an increase in Aβ oligomers accumulated in nerve cells derived from AD(E693Δ)-iPSCs (Figs. 4C and 4D). It was shown that the increase in Aβ oligomers in nerve cells derived from AD-iPSCs can be suppressed by treatment with BSI to achieve a level equivalent to that of the control.

Dot blot analysis was similarly carried out for nerve cells and astrocytes derived from the control iPSCs, AD(E693Δ)-iPSCs, AD(V717L)-iPSCs and sporadic AD-iPSCs. As a result, significant increases in Aβ oligomers could be observed for the nerve cells and astrocytes derived from all cases of AD(E693Δ)-iPSCs and one case of sporadic AD-iPSCs (Figs. 5A, 5B and 5C). Further, it was shown that these increases can be suppressed by treatment with BSI to achieve levels equivalent to the level in the control.

Subsequently, forced expression of the E693Δ type, which is a mutant-type APP, in nerve cells derived from the control iPSCs was carried out. As a result, it could be confirmed that these cells had more Aβ oligomer-positive spot structures as compared to nerve cells derived from iPSCs that had not been subjected to the forced expression (Fig. 5D). Thus, it was suggested that the APP-E693Δ mutation causes accumulation of Aβ oligomers in the cell.

### Example 6

### Search of Other Markers Specific to Alzheimer's Disease

It is known that APP processing by β- and γ-secretase activities proceeds in the vesicle/endosome fraction. In view of this, whether Aβ oligomer-positive spot structures are present in the organelle and vesicle fractions in nerve cells derived from AD(E693Δ)-iPSCs was investigated. As a result, Aβ oligomer-positive spot structures were co-stained with an endoplasmic reticulum (ER) marker BiP, an early-endosome marker EEA1, and a lysosome marker LAMP2 (Fig. 6A). Investigation of the protein expression levels of BiP and activated caspase 4, which are ER stress markers, was carried out. As a result, expression of BiP and activated caspase 4 was found to be higher in nerve cells derived from AD(E693Δ)-iPSCs than in the control (Figs. 6B, 6C and 6D; and Fig. 8).

On the other hand, treatment with a β-secretase inhibitor (BSI), which suppresses production of Ap, suppressed expression of these ER stress markers in nerve cells derived from AD-iPSCs (Figs. 6B and 6C; and Fig. 8). Thus, it is thought that the mutant Aβ of the APP-E693Δ type caused the ER stress.

### Example 7

### Gene Expression Analysis Using DNA Microarray

In order to find molecules differently expressed in nerve cells of the APP-E693Δ type, comprehensive analysis using a DNA microarray was carried out. As a result, increased expression of transgelin was found in AD(E693Δ)-iPSCs (Fig. 7A). Further, gene ontology analysis revealed an enhancement of the oxidative stress-related category including peroxiredoxin activity, oxidoreductase activity and peroxidase activity in nerve cells derived from AD(E693Δ)-iPSCs. On the other hand, the analysis revealed a decline in the glycosylation-related category, suggesting instability of endoplasmic reticulum/Golgi body functions in nerve cells of patients with Alzheimer's disease. In this analysis, expression of PRDX4 (peroxiredoxin-4), which is involved in peroxiredoxin activity, was found to be higher in the nerve cells derived from AD-iPSCs than in the control (Fig. 7B). Measurement by Western blotting revealed that the expression level of PRDX4 is 3-fold increased in the nerve cells derived from AD-iPSCs (Figs. 7C and 7D; and Fig. 8). Since this increase was suppressed by addition of BSI, it was thought that the oxidative stress was caused by formation of Aβ oligomers.

Further, investigation of nerve cells and astrocytes derived from AD(V717L)-iPSCs and sporadic AD-iPSCs revealed increased expression of BiP and peroxiredoxin-4 in nerve cells derived from one case of sporadic AD-iPSCs, similarly to the nerve cells derived from AD(E693Δ)-iPSCs (Fig. 8). Further, astrocytes derived from AD(E693Δ)-iPSCs and one case of sporadic AD-iPSCs showed similar tendencies to those of the above nerve cells in expression of BiP, activated caspase 4 and peroxiredoxin-4 (Fig. 9).

Subsequently, the levels of reactive oxygen species (ROS) in nerve cells and astrocytes derived from AD(E693Δ)-iPSCs, AD(V717L)-iPSCs and sporadic AD-iPSCs were measured by fluorescent staining (HPF method (Sekisui Medical) and CellROX method (Invitrogen)). As a result, increases in the level of reactive oxygen species were found in nerve cells and astrocytes derived from AD(E693Δ)-iPSCs and one case of sporadic AD-iPSCs (Figs. 7E and 7F; Figs. 10A, 10B and 10C; and Fig. 11). Since these increases in the ROS level were also suppressed by addition of BSI, it is thought that the production of ROS was also caused by formation of Aβ oligomers.

### Example 8

### Effect of DHA

Docosahexaenoic acid (DHA) (Nacalai) was added to the medium at concentrations of 1 µM, 5 µM and 15 µM, and the expression levels of BiP protein, activated caspase 4 and PRDX4 in nerve cells derived from AD(E693Δ)-iPSCs were investigated. As a result, it was found that the expression levels were decreased as compared to those in the DHA-free group (DMSO) (Figs. 12A, 12B, 12C and 12D). Further, when iPSC-derived nerve cells were cultured after addition of DHA at 5 µM, reduced expression of ROS was found in nerve cells derived from AD(E693Δ)-iPSCs (Figs. 12E and 12F).

Similarly, as a result of observation of the effect of DHA in nerve cells derived from one case of sporadic AD-iPSCs, significant suppression of the expression levels of ER stress markers (BiP and peroxiredoxin-4) was found similarly to the nerve cells derived from AD(E693Δ)-iPSCs (Figs. 13A and 13B).

On the other hand, addition of DHA did not change the amount of Aβ oligomers accumulated in nerve cells (Fig. 13C).

Thus, in order to investigate the effect of DHA, the cell survival rate was studied for nerve cells derived from AD(E693Δ)-iPSCs and one case of sporadic AD-iPSCs. The cell survival rate of the nerve cells was measured on Day 65 after their induction, by counting EGFP-positive nerve cells prepared by introduction, using a lentivirus, of a vector that expresses EGFP under the Synapsin I promoter (Synapsin::EGFP). Briefly, iPSC-derived nerve cells having EGFP which is induced under the Synapsin I promoter (Synapsin::EGFP) were transferred onto a Matrigel coat, and 5 days later, the medium was replaced with a DHA-containing or DHA-free medium which is free of B27 and neurotrophic factors (BDNF, GDNF and NT-3). From Hour 48, the number of EGFP-positive cells was measured every hour using an IN CELL Analyzer 2000 (Figs. 14A and 14B). In this process, nerve cell death can be determined by observation of loss of EGFP, destruction of the cell membrane, blebbing and/or the like. The nerve cell survival rate was calculated based on the ratio to the initially counted number. Cell survival was also studied using the lactate dehydrogenase (LDH) method. The LDH method was carried out by measuring the LDH level in the cell culture liquid using a Cytotoxicity Detection Kit (LDH) (Roche Diagnostics) (Fig. 14C). In the LDH method, the number of dead cells was evaluated as the ratio to that of Conterol-1. As a result, it could be confirmed that, in the nerve cells derived from AD(E693Δ)-iPSCs, cell death caused by the absence of neurotrophic factors can be suppressed by DHA. Effectiveness of DHA could be confirmed also for cell death caused by hydrogen peroxide.

The above results are summarized below in Table 1.

### [Table 1]

**Table 1**

| Candidate agent | Aβ oligomer | ER stress markers (BiP, Peroxiredoxin-4, etc.) |
|---|---|---|
| β-Secretase inhibitor IV (BSI) | Decreased | Decreased |
| Docosahexaenoic (DHA) | Unchanged | Decreased |

That is, when Aβ oligomers is used as an index, DHA cannot be selected as a candidate for the agent, but at least BSI can be selected.

Thus, it could be confirmed that BSI or DHA can be found to be a possible candidate for a therapeutic and/or prophylactic agent for Alzheimer's disease by using as an index the Aβ oligomer level; an ER stress marker BiP or activated caspase 4; a peroxiredoxin activity-related gene PRDX4; or intracellular ROS.

### INDUSTRIAL APPLICABILITY

The present invention is based on the fact that the disease state of Alzheimer's disease could be reproduced in nerve cells whose differentiation was induced from iPS cells derived from somatic cells of a patient with Alzheimer's disease. Therefore, the cells can be used for screening of a therapeutic and/or prophylactic agent for Alzheimer's disease.

## Claims

1. A method for screening a therapeutic agent and/or prophylactic agent for Alzheimer's disease, said method comprising the steps of:
(a) bringing a candidate substance into contact with a astrocyte(s) derived from an induced pluripotent stem (iPS) cell(s) prepared from a somatic cell(s) of a patient with Alzheimer's disease, or derived from an iPS cell(s) in which amyloid precursor protein having deletion mutation of glutamic acid at position 693 has been introduced;
(b) measuring the amount of amyloid β oligomers in said astrocyte(s); and
(c) selecting said candidate substance as a therapeutic and/or prophylactic agent for Alzheimer's disease if the amount of amyloid β oligomers is decreased as compared to a case where said candidate substance is not brought into contact.

2. The method according to claim 1, wherein said astrocyte is a cell that accumulates amyloid β oligomers.

3. The method according to claim 1 or 2, wherein said somatic cell of a patient with Alzheimer's disease is a somatic cell having amyloid precursor protein having deletion mutation of glutamic acid at position 693.

## Patentansprüche

1. Verfahren zum Screening eines therapeutischen Agens und/oder prophylaktischen Agens für Alzheimer-Erkrankung, wobei das Verfahren die Schritte umfasst:
(a) Inkontaktbringen einer Auswahlsubstanz mit (einer) Astrozyte(n), abgeleitet aus einer induzierten pluripotenten Stammzelle(n) (iPS-Zellen), welche aus einer somatischen Zelle(n) eines Patienten mit Alzheimer-Erkrankung hergestellt wurde, oder abgeleitet aus einer iPS-Zelle(n), in welche Amyloid-Vorläuferprotein mit Deletionsmutation von Glutaminsäure an Position 693 eingebracht wurde;
(b) Messen der Menge an Amyloid-β-Oligomeren in der/n Astrozyte(n); und
(c) Auswählen der Auswahlsubstanz als ein therapeutisches Agens und/oder prophylaktisches Agens für Alzheimer-Erkrankung, wenn die Menge an Amyloid-β-Oligomeren verringert ist im Vergleich zu einem Fall, wo die Auswahlsubstanz nicht in Kontakt gebracht wird.

2. Verfahren gemäß Anspruch 1, wobei die Astrozyte eine Zelle ist, welche Amyloid-β-Oligomere anreichert.

3. Verfahren gemäß Anspruch 1 oder 2, wobei die somatische Zelle eines Patienten mit Alzheimer-Erkrankung eine somatische Zelle mit Amyloid-Vorläuferprotein mit Deletionsmutation von Glutaminsäure an Position 693 ist.

## Revendications

1. Procédé de criblage d'un agent thérapeutique et/ou d'un agent prophylactique pour la maladie d'Alzheimer, ledit procédé comprenant les étapes :
(a) de mise en contact d'une substance candidate avec un/des astrocyte(s) dérivé(s) d'une/des cellule(s) souche(s) pluripotente(s) induite(s) (iPS) préparée(s) à partir d'une/des cellule(s) somatique(s) d'un patient atteint de la maladie d'Alzheimer, ou dérivée(s) d'une/des cellule(s) iPS dans laquelle/lesquelles une protéine précurseur de l'amyloïde ayant une mutation de type délétion d'un acide glutamique en position 693 a été introduite ;
(b) de mesure de la quantité d'oligomères de peptide β-amyloïde dans ledit/lesdits astrocyte(s) ; et
(c) de la sélection de ladite substance candidate comme agent thérapeutique et/ou prophylactique de la maladie d'Alzheimer si la quantité d'oligomères de peptide β-amyloïde est réduite par rapport à un cas dans lequel ladite substance candidate n'est pas mise en contact.

2. Procédé selon la revendication 1, dans lequel ledit astrocyte est une cellule qui accumule des oligomères de peptide β-amyloïde.

3. Procédé selon la revendication 1 ou 2, dans lequel ladite cellule somatique d'un patient atteint de la maladie d'Alzheimer est une cellule somatique ayant une protéine précurseur de l'amyloïde ayant une mutation de type délétion d'un acide glutamique en position 693.
